# EUROPEAN PATENT SPECIFICATION

(11) **EP 3 847 244 B1**
(45) Date of publication and mention of the grant of the patent: **30.07.2025**
(21) Application number: 19858191.0
(22) Date of filing: 06.09.2019
(51) Int. Cl.: C12N 5/0789, C12N 5/0797, A61L 27/38, A61K 35/30, C12N 5/0793, G01N 33/50

(54) **METHODS AND COMPOSITIONS FOR RETINAL NEURON GENERATION IN CARRIER-FREE 3D SPHERE SUSPENSION CULTURE**
VERFAHREN UND ZUSAMMENSETZUNGEN ZUR RETINALEN NEURONENERZEUGUNG IN EINER TRÄGERFREIEN 3D-KUGELSUSPENSIONSKULTUR
PROCÉDÉS ET COMPOSITIONS POUR LA GÉNÉRATION DE NEURONES RÉTINIENS DANS UNE CULTURE EN SUSPENSION DE SPHÈRES 3D SANS SUPPORT

(30) Priority: 07.09.2018 US 201862728088 P
(43) Date of publication of application: 14.07.2021
(73) Proprietor: Hebecell Corporation, Natick, MA 01760 (US)
(72) Inventor: QIANG, Feng, Natick, Massachusetts 01760 (US); ELSEN, Gina, Natick, Massachusetts 01760 (US); LU, Shi-Jiang, Natick, Massachusetts 01760 (US)
(74) Representative: Mewburn Ellis LLP
(86) International application number: PCT/US2019/049916
(87) International publication number: WO 2020/051429

(56) References cited:
- WO-A1-2015/121687
- US-A1- 2016 030 490
- PATRICK OVANDO-ROCHE ET AL: "Use of bioreactors for culturing human retinal organoids improves photoreceptor yields", STEM CELL RESEARCH & THERAPY, vol. 9, no. 1, 13 June 2018 (2018-06-13), pages 1 - 14, XP055693039, DOI: 10.1186/s13287-018-0907-0
- KARL J. WAHLIN ET AL: "Photoreceptor Outer Segment-like Structures in Long-Term 3D Retinas from Human Pluripotent Stem Cells", SCIENTIFIC REPORTS, vol. 7, no. 1, 10 April 2017 (2017-04-10), XP055598785, DOI: 10.1038/s41598-017-00774-9
- ANAI GONZALEZ-CORDERO ET AL: "Recapitulation of Human Retinal Development from Human Pluripotent Stem Cells Generates Transplantable Populations of Cone Photoreceptors", STEM CELL REPORTS, vol. 9, no. 3, 1 September 2017 (2017-09-01), United States, pages 820 - 837, XP055466167, ISSN: 2213-6711, DOI: 10.1016/j.stemcr.2017.07.022
- TOKUSHIGE NAKANO ET AL: "Self-Formation of Optic Cups and Storable Stratified Neural Retina from Human ESCs", CELL STEM CELL, ELSEVIER, CELL PRESS, AMSTERDAM, NL, vol. 10, no. 6, 14 June 2012 (2012-06-14), pages 771 - 785, XP028492663, ISSN: 1934-5909, DOI: 10.1016/J.STEM.2012.05.009
- FENG QIANG: "Robust Generation of Photoreceptor Precursors from Human Pluripotent Stem Cells Using a Scalable 3D Bioreactor System | IOVS | ARVO Journals", ARVO ANNUAL MEETING ABSTRACT, 1 July 2019 (2019-07-01), XP055955532, Retrieved from the Internet <URL:https://iovs.arvojournals.org/article.aspx?articleid=2741009> [retrieved on 20220829]
- WILEY , LA ET AL.: "cGMP Production of Patient-Specific iPSCS and Photoreceptor Precursor Cells to Treat Retinal Degenerative Blindness", SCIENTIFIC REPORTS, vol. 6, no. 30742, 29 July 2016 (2016-07-29), pages 1 - 16, XP055693035
- VOLKNER, M ET AL.: "Retinal Organoids from Pluripotent Stem Cells Efficiently Recapitulate Retinogenesis", STEM CELL REPORTS, vol. 6, no. 4, 31 March 2016 (2016-03-31), pages 525 - 538, XP055372696, DOI: 10.1016/j.stemcr.2016.03.001
- OVANDO-ROCHE, P ET AL.: "Use of Bioreactors for Culturing Human Retinal Organoids Improves Photoreceptor Yields", STEM CELL RESEARCH AND THERAPY, vol. 9, no. 1, 13 June 2018 (2018-06-13), pages 1 - 14, XP055693039

## Description

### CROSS REFERENCE TO RELATED APPLICATIONS

This application claims priority to and the benefit of U.S. Provisional Patent Application No. 62/728,088 filed September 7, 2018.

### FIELD

The present disclosure relates generally to methods and compositions for retinal neuron generation from, e.g., human pluripotent stem cells.

### BACKGROUND

Age-related macular degeneration and inherited retinal degenerations represent the leading causes of untreatable blindness in the developed world [1]. They share a common final pathophysiology, the loss of the light sensitive photoreceptors, which consists of rods and cones. Replacement of lost photoreceptors may offer a potential treatment strategy for both patient populations, who lack effective treatment options [1-3]. Most macular diseases involve the loss of both photoreceptors and retinal pigment epithelium (RPE), the latter support and maintain the health of photoreceptor in the outer retina. Transplantation of human embryonic stem cell (hESC)-derived RPE to treat patients with dry age-related macular degeneration (AMD) and Stargardt's disease in clinical trials have been published and shown evidence of safety and efficacy including an assessment after four years [4-6], but no such study has been carried out for photoreceptors derived form human pluripotent stem cells (hPSC) in human clinical trial [7].

The main requirement for developing effective photoreceptor transplantation therapies is the establishment of robust protocols that permit the generation of a large quantity of homogenous photoreceptor precursor cells (PRPC) from renewable sources such as hESCs and human induced pluripotent stem cells (hiPSC), since PRPCs form human tissues are limited in supply with major ethical barriers [8]. During the past decades, there have been remarkable progress in the ability to generate retinal cell types with various degrees of efficiency, including PRPCs and photoreceptors, from a variety of murine and human PSC sources in *vitro,* and the potentials of hPSC-derived PRPCs have been established in pre-clinical transplantation studies with different animal models [2, 7, 9-13]. Currently, most differentiation protocols utilized conventional adherent two-dimensional (2D) planar cultures which are commonly initiated differentiation directly as a 2D monolayer by exposure to a cocktail of protein factors [9-11, 14, 15], or uncontrolled formation of cellular aggregates known as embryoid bodies (EBs) in static cultures which were later attached to coated or non-coated surfaces for further differentiation [1, 2, 8]. Recently, Sasai and colleagues described an EB-based self-organizing 3D differentiation system called retinal organoids which recapitulates many aspects of normal retinal development [16]. Based on this technique, PRPCs and photoreceptors have been generated from human PSC-laminated 3D retinal organoids by several laboratories [12, 17-21]. However, the long-term cultured organoids contain numerous cell types at different developmental stages and formation of tight junctions between these cell types, which render them fragile to enzymatic or physical dissociation with the generation of unhealthy and heterogeneous cell populations. In addition, although all these approaches generated photoreceptors or their progenitors which can be used for research purposes, they are not suitable to generate transplantable cells for clinical applications due to the use of undefined proteins of non-human origin, which present major safety concerns. WO 2015/121687 reports methods of producing synthetic retina using 3D stem cell culture. Ovando-Roche et al Stem Cell Research & Therapy 9 1 (2018) 1-14 reports the use of bioreactors to culture human retinal organoids. Wahlein et al Scientific Reports 7 1 (2017) reports the production of long-term 3D retinas from human pluripotent cells. Gonzalez-Cordero et al: Stem Cell Reports,9 3 (2017) 820-837 reports the generation of transplantable populations of cone photoreceptors from human pluripotent stem cells. Nakano et al Cell Stem Cell 10 6 (2012) 771-785 reports the self-formation of optic cups and storable stratified neural retina from human ESCs.
Thus, a need exists for scalable platforms capable of generating homogenous populations of specific cells from hPSCs, including PRPCs and photoreceptors, under defined conditions.

### SUMMARY

The present disclosure provides, *inter alia,* a method for in vitro production of photoreceptor precursor cells, comprising:
(a) 3-dimensional (3D) sphere culturing a plurality of pluripotent stem cells to generate a plurality of first spheres comprising eye early and late committed retinal neural progenitors (CRNPs);
(b) monitoring sphere size until the first spheres reach an average size of about 300-500 µm in diameter;
(c) disassociating the first spheres into a first plurality of substantially single cells;
(d) 3D sphere culturing the first plurality of substantially single cells to generate a plurality of second spheres comprising photoreceptor precursor cells (PRPCs);
(e) monitoring sphere size until the second spheres reach an average size of about 300-500 µm in diameter;
(f) disassociating the second spheres into a second plurality of substantially single cells;
(g) 3D sphere culturing the second plurality of substantially single cells to generate a plurality of third spheres comprising postmitotic PRPCs; and
(h) optionally, further differentiating the postmitotic PRPCs into photoreceptor-like cells.

In some embodiments, the pluripotent stem cells can be embryonic stem cells or induced pluripotent stem cells, preferably from human.

In some embodiments, steps (a), (d) and (g) comprise culturing in a spinner flask or a stir-tank bioreactor, preferably under continuous agitation.

In some embodiments, step (a) further comprises gradually adapting to and culturing in a neural induction medium, preferably NIM-3D (Neural Induction Medium-3D) basal medium containing DMEM/F12 with HEPES, N2 and B27 serum-free supplements, penicillin/streptomycin, MEM non-essential amino acids, and glucose, supplemented with one or more of Sonic Hedgehog, Heparin, IWR-1e, SB431542, LDN193189 and IGF1.

In some embodiments, the method can further include providing SB431542, LDN193189 and IGF1 in the neural induction medium for a first period of time, providing IWR-1e in the neural induction medium for a second period of time that is shorter than the first period of time, and providing Sonic Hedgehog or Heparin in the neural induction medium for a third period of time that is shorter than the second period of time. In some embodiments, the first period of time is 10-20 days, preferably 12-18 days, more preferably 16 days. In some embodiments, the second period of time is 5-15 days, preferably 8-14 days, more preferably 11 days. In some embodiments, the third period of time is 3-12 days, preferably 5-10 days, more preferably 7 days.

In some embodiments, Sonic Hedgehog or Heparin can be withdrawn during neural commitment, e.g., about 1-5 days, 2-4 days or 2 days prior to IWR-1e withdrawal. IWR-1e can be withdrawn upon complete adaptation, about 1-5 days (or 2-4 days or 1 day) prior to complete adaptation, in the neural induction medium. Complete adaption in a certain medium as used herein refers to culturing in 100% such medium. SB431542, LDN193189 and IGF1 can be withdrawn upon initiation of, or 1-5 days (or 2-4 days or 3 days) prior to, adaptation into a photoreceptor differentiation medium.

In some embodiments, in step (b) the first spheres can reach an average size of about 350-450 µm in diameter. In some embodiments, in step (b) the first spheres can reach an average size of less than about 400 µm in diameter.

In some embodiments, steps (c) and (f) comprise contacting the first spheres and the second spheres, respectively, with a cell-dissociation enzyme.

In some embodiments, step (d) further comprises gradually adapting to and culturing in a photoreceptor differentiation medium, preferably PRPC-3D medium containing Neurobasal^{™} medium, N2 and B27 serum-free supplements, penicillin/streptomycin, MEM non-essential amino acids, and glucose.

In some embodiments, step (g) and/or (h) further comprises switching to and culturing in a maturation medium, preferably Neurobasal^{™} medium containing L-glutamine (e.g., GlutaMAX^{™}), Penicillin/streptomycin, human brain-derived neurotrophic factor (BDNF), ascorbic acid, and DAPT (N-[N-(3,5-difluorophenacetyl)-1-alanyl]-S-phenylglycine t-butyl ester). In certain embodiments, said switching can include gradually adapting into the maturation medium.

In some embodiments, step (g) and/or (h) further comprises monitoring sphere size until about 300-500 µm in diameter; disassociating the third spheres into a third plurality of substantially single cells, preferably with a cell-dissociation enzyme; and reaggregating the third plurality of substantially single cells.

Other disclosure relates to a neural induction medium comprising DMEM/F12 with HEPES, N2 and B27 serum-free supplements, penicillin/streptomycin, MEM non-essential amino acids, glucose, and one or more of Sonic Hedgehog, Heparin, IWR-1e, SB431542, LDN193189 and/or IGF1.

Other disclosure relates to a maturation medium comprising Neurobasal^{™} medium, L-glutamine (e.g., GlutaMAX^{™}), Penicillin/streptomycin, human brain-derived neurotrophic factor (BDNF), ascorbic acid, and DAPT (N-[N-(3,5-difluorophenacetyl)-1-alanyl]-S-phenylglycine t-butyl ester).

### BRIEF DESCRIPTION OF THE DRAWINGS

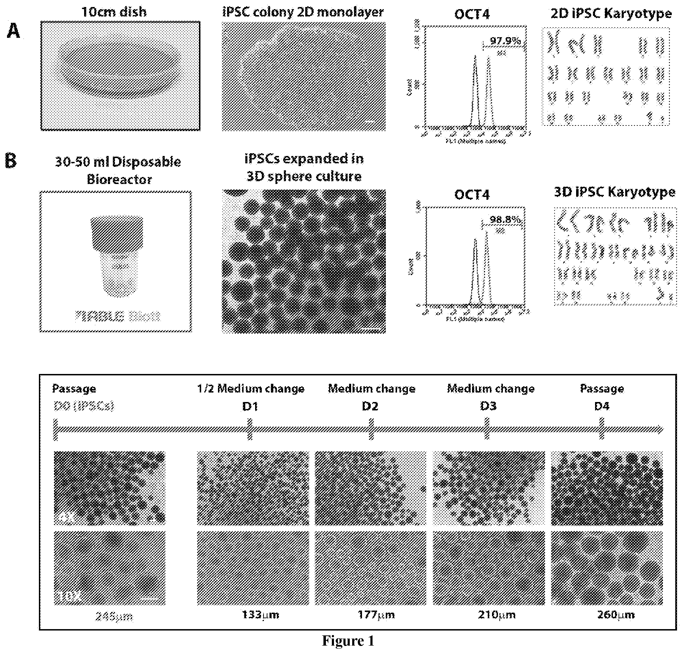

Figure 1: Characterization of 2D monolayer and 3D sphere hiPSCs. (Panel A) From left to right, a 10 cm 2D-cell culture dish, hiPSC monolayer colony, flow cytometry histogram of OCT4 positive hiPSCs (97.9%) and karyotype analysis of hiPSCs from 2D culture. (Panel B) (Upper, from left to right) A spinner flask, 3D hiPSC spheres cultured in a 30ml spinner (bioreactor), flow cytometry histogram of OCT4 positive hiPSCs (98.8%) and karyotype analysis of hiPSCs from 3D spheres. (Bottom) A typical 3D sphere passage cycle, medium change, morphology and sphere size from one hiPSC line during the 4-day passage interval.

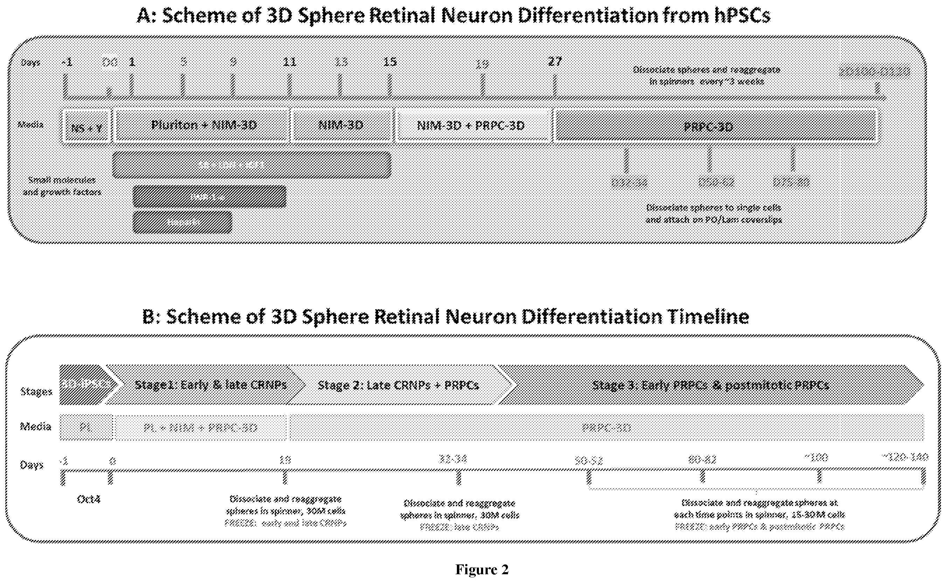

Figure 2: 3D sphere differentiation protocol and timeline. (Panel A) Scheme shows 3D sphere PRPC differentiation over a period of about 100 days. The days of differentiation, specific media and small molecules and growth factors used at each time point are indicated. (Panel B) Scheme shows the stepwise strategy used for the induction and differentiation of 3D-hiPSCs into eye early and late committed retinal neural progenitors (CRNP) and photoreceptor precursor cells (PRPC). Timepoints for freezing cells are indicated in the diagram.

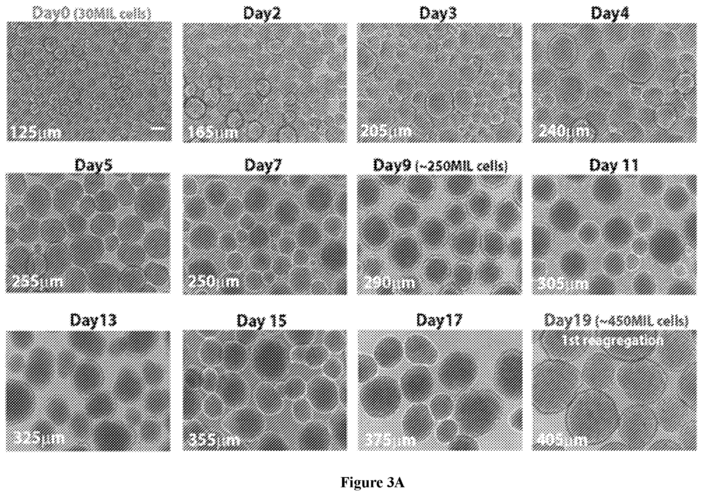

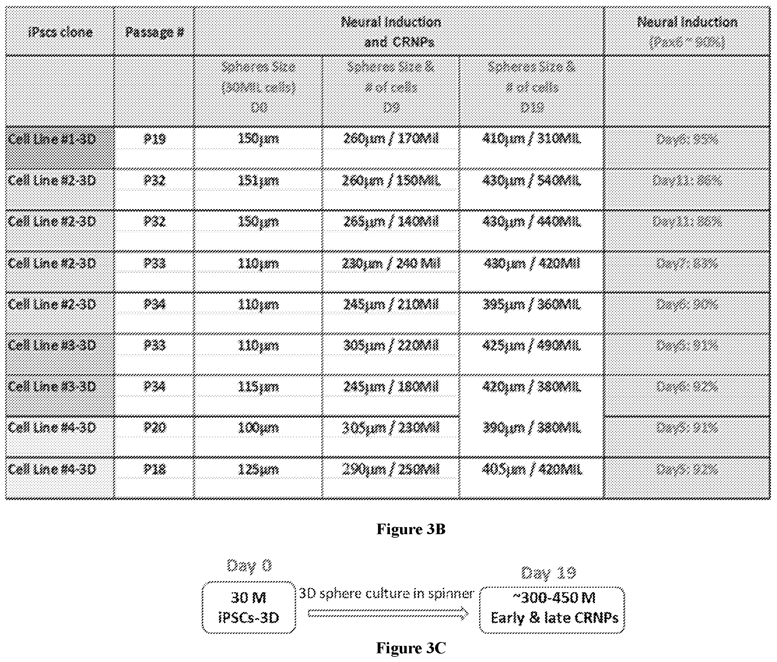

Figures 3A-3C: Induction of committed retinal neuronal progenitors in 3D spheres from hiPSCs. Figure 3A: Phase contrast images show the intact morphology and expansion of 3D neural spheres from D0 to D19 in 30 ml spinner flasks. The number of cells at D0 (30 millions), D9 (about 250 millions), and D19 (about 450 millions) are indicated. Figure 3B: Table summarizes results from 4 hiPSC lines used for retinal neuronal differentiation. The efficiency of neural differentiation was assessed by flow cytometry using PAX6 antibody as a marker of neural cells. Figure 3C: Approximate yield of early and late committed retinal neural progenitors (CRNPs) at day 19 generated from one spinner flask starting with 30 million hiPSCs.

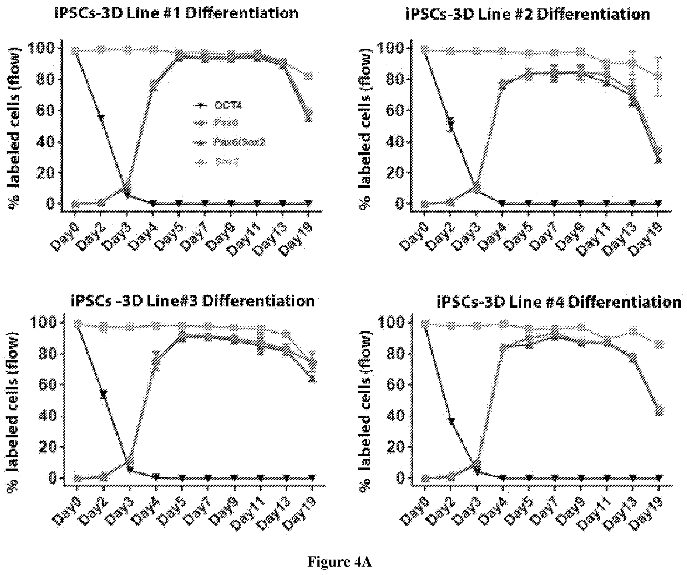

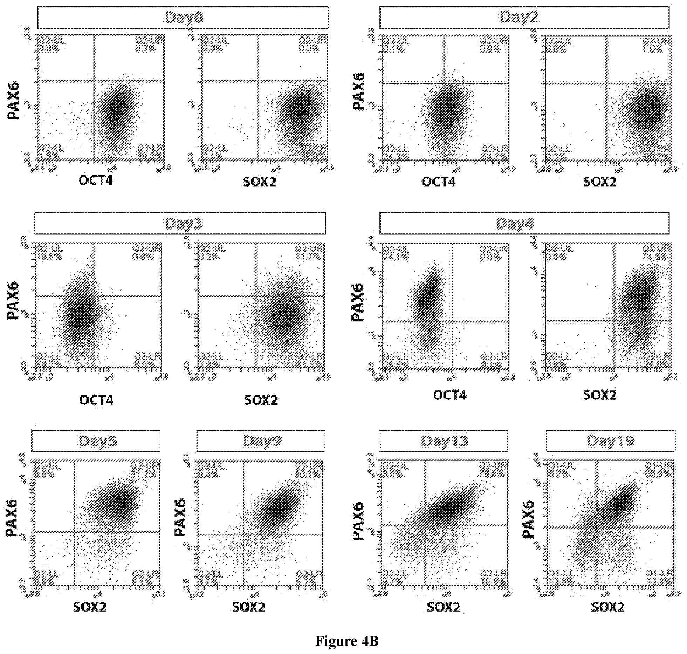

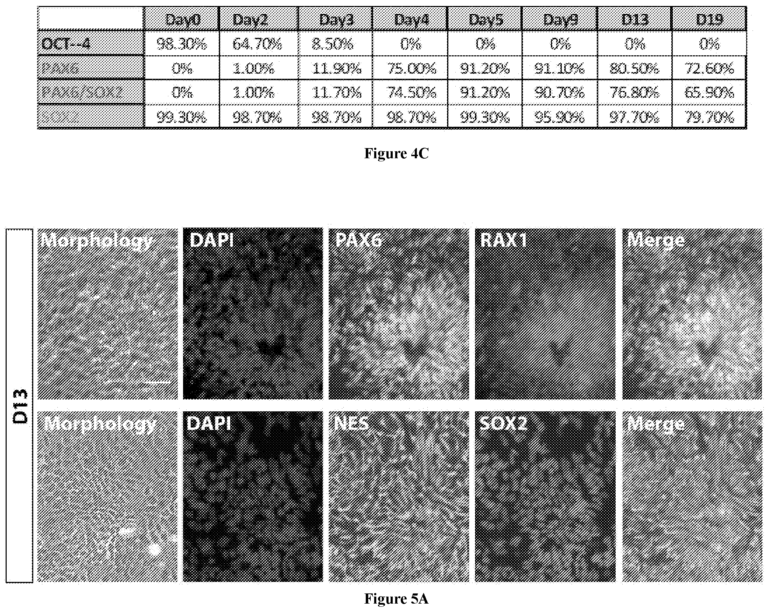

Figures 4A-4C: Expression kinetics of pluripotent and neural markers during induced neuronal differentiation of 3D-hiPSC spheres. Figure 4A: Graphs illustrate the percentage of OCT4, PAX6, PAX6/SOX2, and SOX2-positive cells of four hiPSC lines during differentiation from D0 to D19 analyzed by flow cytometry. Figure 4B: Flow cytometry analyses of OCT4, PAX6, PAX6/SOX2 positive cells at different days of neural differentiation. Figure 4C: Table summarizes results from flow cytometry analyses.

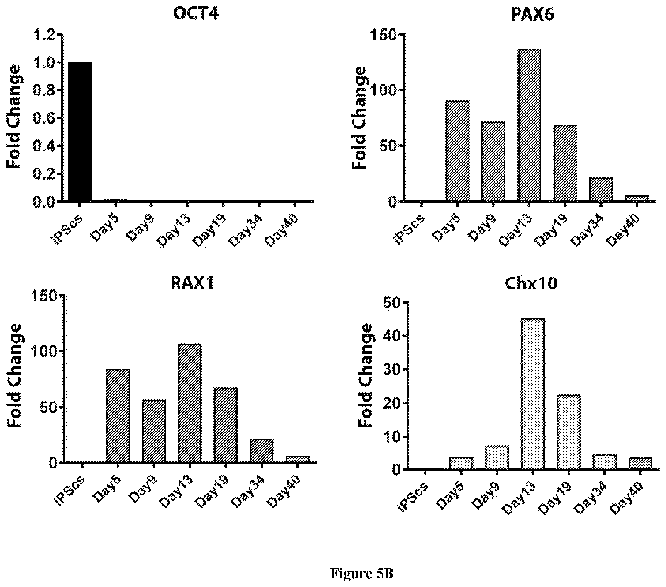

Figures 5A-5B: Characterization of neural markers in 3D sphere cells. Figure 5A: Phase contrast images and immunofluorescence staining show PAX6, RAX1, NESTIN and SOX2 on cells derived from 3D neural spheres. Differentiated cells at D5 were plated and cultured for 8 days, then stained with antibodies for neural specific marker PAX6 (green), CRNP marker RAX1 (red), and general neural markers NESTIN (green) and SOX2 (red). A high percentage of cells were double positive for PAX6/RAX1 and NES/SOX2 indicating the acquisition of neural and early and late CRNP identity. Nuclei were counterstained with DAPI. Scale bar, 100 µm. Figure 5B: Quantification analyses of gene expression by qRT-PCR at different days of induced differentiations. The expression of OCT4 disappeared completely by D5; PAX6 expression was elevated at D5 and continued to D19, then followed by a gradual decrease until D40; RAX1 expression showed a similar pattern of PAX6; CHX10 expression showed a peak at D13 followed by decrease at D40, which suggests acquisition of early and late CRNP phenotype during this period of differentiation and initiation of PRPC specification post D40.

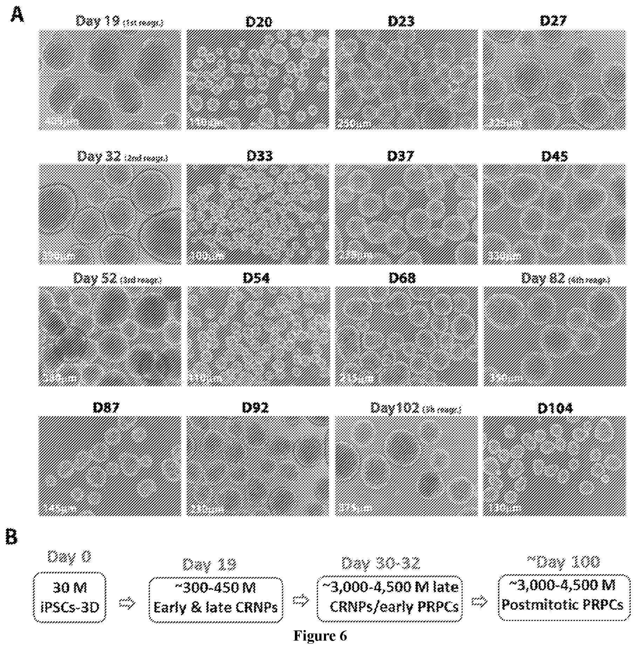

Figure 6: Retinal neuron spheres generated by continuous dissociation/reaggregation and sphere reformation regimen. (Panel A) Phase contrast images show morphologies and expansion patterns of 3D retinal neuron spheres at different stages of hiPSC differentiation in 30 ml spinner flasks. Scale bar =100 µm. (Panel B) Schematic summary illustrates yields of early and late CRNPs and postmitotic PRPCs from 30 million starting 3D-hiPSCs.

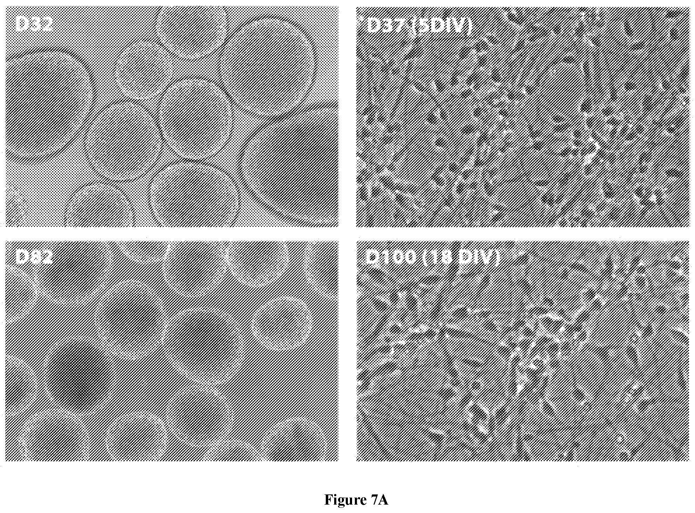

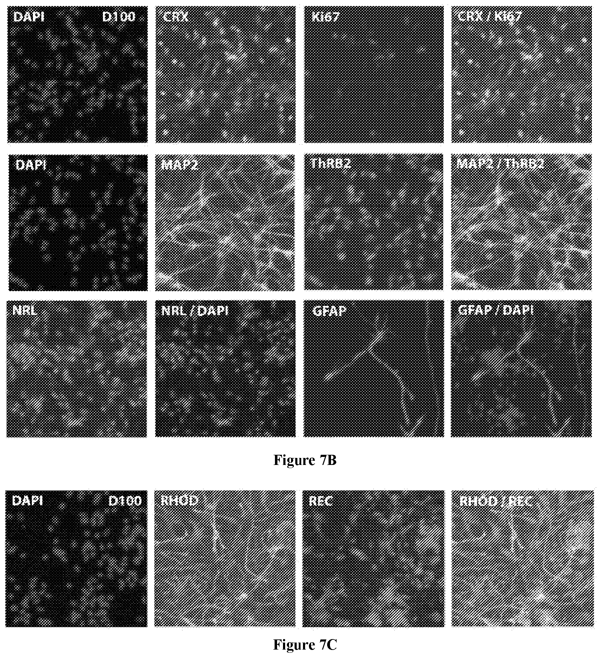

Figures 7A-7C: Characterization of 3D-PRPCs generated from hiPSCs. Figure 7A: Phase contrast images show 3D hiPSCs-derived 3D neural sphere morphologies prior to dissociation at D32 (top) and D82 (bottom) and dissociated single cells cultured on surface for 5 days (D37) and 18 days (D100), respectively. Figure 7B: Immunofluorescence staining shows expression of CRX (green), ThRB2 (red), and NRL (red), MAP2 (green), and GFAP (red) on cells derived from 3D-PRPC spheres at D82 cultured on surface for additional 18 days. Ki67 staining (red) shows a very low percentage of mitotic cells at D100. DAPI is used for nuclear staining. Figure 7C: Immunofluorescence staining shows expression of photoreceptor specific protein rhodopsin (RHOD, green) and recoverin (REC, red) on cells derived from 3D-PRPC spheres at D82 cultured on surface for additional 18 days. DAPI is used for nuclear staining.

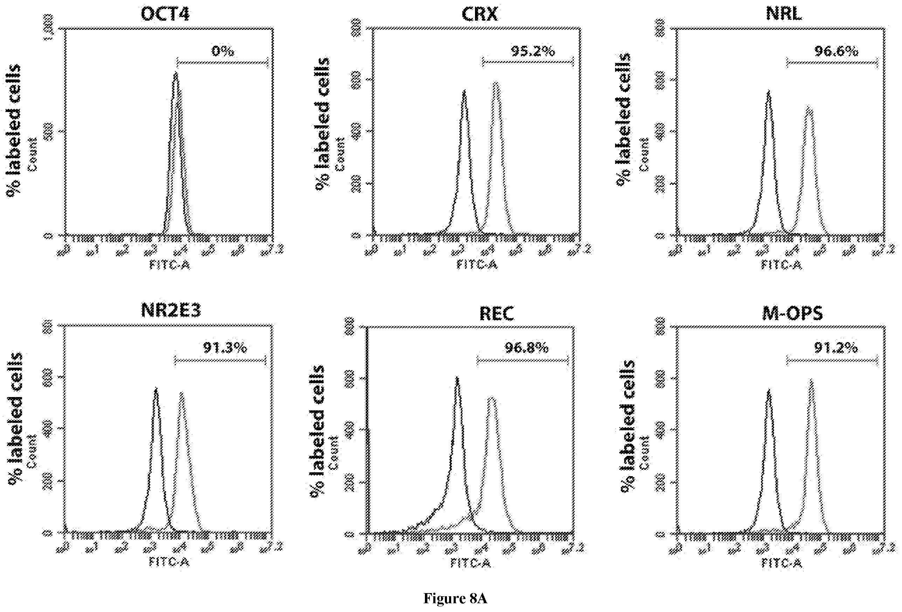

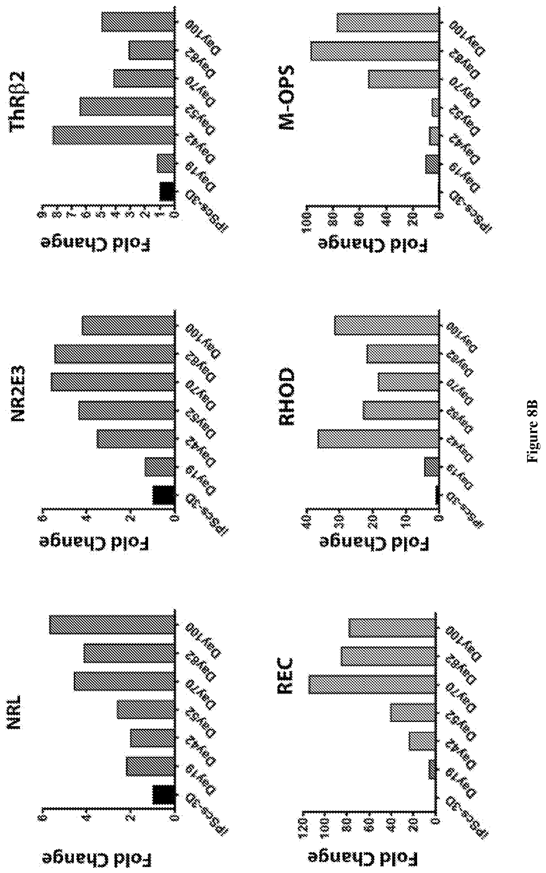

Figures 8A-8B: Molecular characterization of 3D-PRPCs derived from hiPSCs. Figure 8A: Quantification of intracellular staining of OCT4 (0%), CRX (95.2%), NRL (96.6%), NR2E3 (91.3%), REC (96.8), and M-Opsin (91.2%) as determined by Flow Cytometry analyses in 3D spheres cells after 80 days of differentiation. Secondary antibody only was used as negative control. Figure 8B: Quantitative RT-PCR analyses of PRPC and photoreceptor markers on 3D sphere cells obtained from different days of retinal neural differentiation, which show a gradual acquisition of PRPC phenotype (NRL, NR2E3, ThRb2) and photoreceptor characteristics (REC, RHOD, M-Opsin).

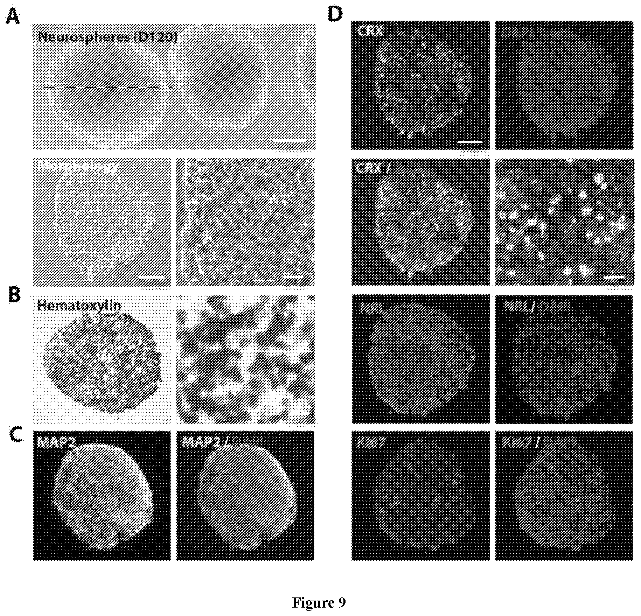

Figure 9: Characterization of cells inside 3D spheres after 120 days of differentiation. (Panel A) Schematic illustration indicates how the 3D-spheres at D120 were sectioned (interrupted line). Phase contrast images indicate intact morphology and integrity at the central core of spheres. Scale bar, 100 µm. (Panel B) Hematoxylin staining of sections indicates viable cells across the sections. (Panel C) Immunohistochemistry staining on sections of 3D-PRPC spheres at D120 of differentiation shows neuronal marker MAP2 expression. DAPI was used to counterstain nuclei. (Panel D) Immunohistochemistry staining on sections of 3D-spheres at D120 of differentiation shows PRPC markers CRX (green) and NRL (red) expression; and cell proliferating marker Ki67 (red) staining shows very low percentage of proliferation cells in these spheres.

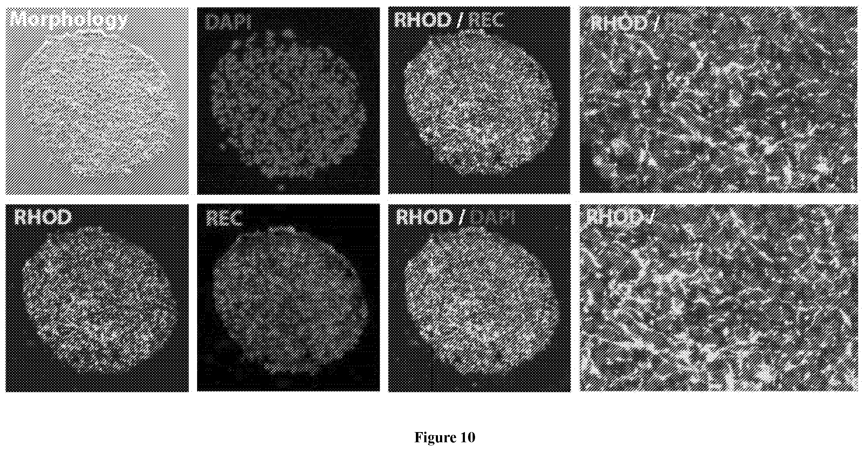

Figure 10: 3D Spheres after 120-day differentiation express markers of photoreceptors. Immunohistochemistry staining on sections of 3D-spheres at D120 of differentiation shows expression photoreceptor markers rhodopsin (RHOD, green) and recoverin (REC, red). DAPI was used to counterstain nuclei.

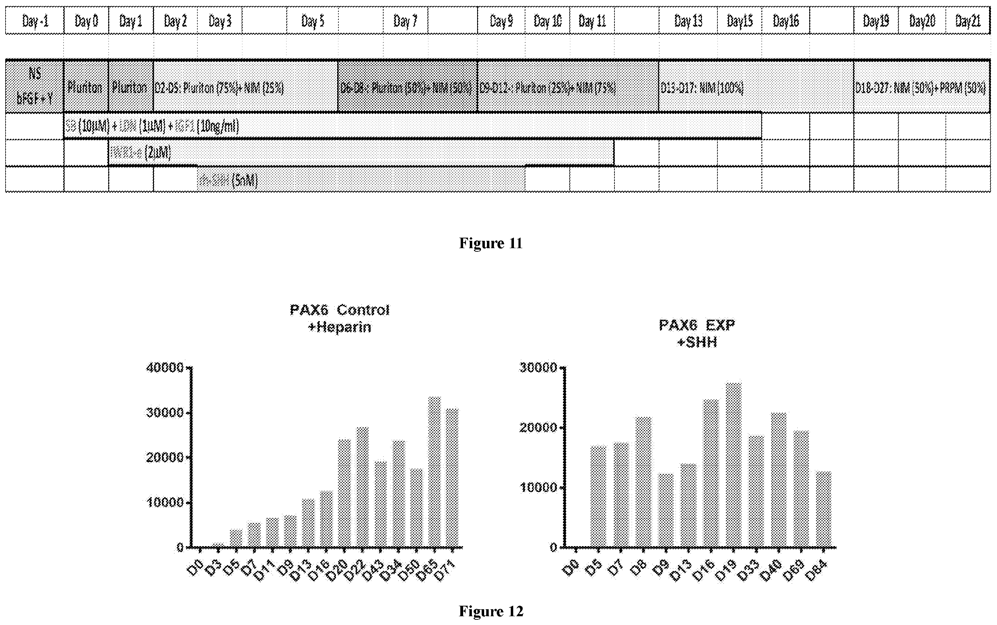

Figure 11: Overview of an exemplary neural induction protocol for the derivation of retinal neural progenitors from human induced pluripotent stem cells. The use of small molecules in combination with Sonic Hedgehog (SHH) efficiently generates retinal progenitor cells.
Figure 12: Comparative RT-PCR quantitation of PAX6 mRNA gene expression in differentiated cells treated with Heparin or SHH. Total RNA was collected from cells in both conditions during the photoreceptor differentiation timeline and analyzed for PAX6 RNA transcript levels.

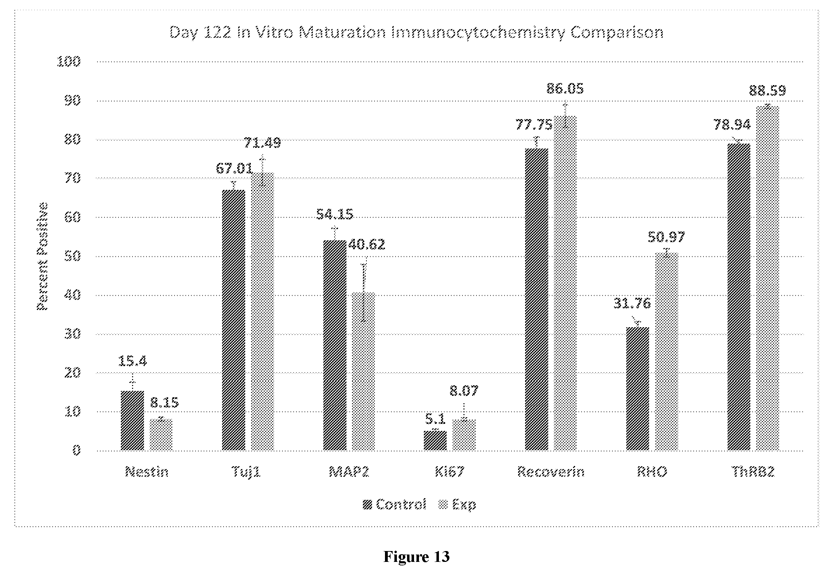

Figure 13: Characterization of differentiation day 122 photoreceptor cells derived from human induced pluripotent stem cells in a modified maturation medium.

### DETAILED DESCRIPTION

Provided herein, in one aspect, is a population of retinal neurons including photoreceptor precursor cells (PRPCs) generated *in vitro* from human pluripotent stem cells (hPSCs) that can be used as a cell source for regenerative therapies, drug discovery and disease modeling. Methods and compositions for making and using the same are also provided.

An essential requirement for the development of cell-based therapies is the establishment of robust manufacture process that allow the derivation of large quantities of highly pure transplantable cells from renewable sources, which recapitulate the characteristics of the endogenous cell types intended to replace. However, numerous approaches to differentiate hPSCs into retinal neurons and PRPCs for the purpose of cell replacement therapy produced undesirable results in terms of efficiency, purity, homogeneity and scalability. Disclosed herein, in one aspect, is a robust, defined and scalable 3-dimensional (3D) sphere culture system for the generation of highly enriched retinal neurons at different developmental stages from hPSCs, including early and late committed retinal neuron progenitors (CRNP), PRPCs as well as photoreceptor-like cells by synchronizing the differentiation process, which can be easily adapted to current general manufacture practice (cGMP) protocol.

In some embodiments, the protocol or process can start with hPSCs as 3D spheres, which are directly induced to differentiate into early CRNPs, late CRNPs, PRPCs and photoreceptor-like cells in a defined, serum-free culture medium. In some embodiments, the culture medium can include a combination of small molecules that can be introduced therein at specified time points during culturing, to induce differentiation, with continuous sphere dissociation/reaggregation and sphere reformation approach in spinner bioreactors under matrix- and carrier-free conditions. This well controlled 3D sphere system overcomes numerous limitations, especially the scalability, facing conventional surface-adherent 2D culture and traditional embryoid body as well as organoid systems. It has been surprisingly discovered that this approach routinely generates 3-4.5 x 10⁹ PRPCs starting with 3 x 10⁶ hiPSCs with a purity of approximately 95%. Multiple levels of analyses, including immunofluorescence staining, flow cytometry, and quantitative gene expression by RT-qPCR confirmed the identities of early and late CRNPs, PRPCs and photoreceptor-like cells generated using this system. Thus, this 3D sphere platform is amenable to the development of an *in vitro* GMP-compliant retinal cell manufacturing protocol from multiple renewable hPSC sources for future preclinical studies and human cell replacement therapies.

A 3D scalable sphere culture system in a defined minimal culture condition can offer a variety of benefits such as scalability, reproducibility and homogenous microenvironments as well as cost effectiveness. In addition, the development of protocols aimed at the generation of enriched retinal neuron populations such as PRPCs at the correct stage is the key to the success of cell replacement therapies, such as cell replacement treatment for photoreceptor lost patients. In some embodiments, the methods disclosed herein mimick the chemical and cytokine microenvironments of signals known to guide retinal histogenesis during normal development, we developed a defined, continuous matrix-and carrier-free 3D sphere culture system with the supplement of small molecules to promote retinal neuron differentiation directly from 3D sphere-adapted hPSCs. The stepwise induced differentiation protocol disclosed herein, in some embodiments, includes regular sphere dissociation/reaggregation at every passage for sphere reformation over a period of about 50-100 days, which lead to formation of uniformed spheres having a controlled, desirable size. Without wishing to be bound by theory, it is believed that the sphere size is important in allowing sufficient penetration of oxygen, nutrients and other factors throughout the spheres, enrichment of neuronal populations and synchronization of neuron differentiation. Furthermore, hPSC spheres differentiated under this protocol sequentially acquire markers specific for neural cells (PAX6), early and late committed retinal neuron progenitors (RAX1 and CHX10), PRPCs (CRX, NRL, NR2E3, ThRB2), and photoreceptors (REC, RHOD and M-OPSIN), with a purity of about 95%.

Significantly, provided herein is methodology for the integration of undifferentiated hPSC expansion and streamlined small molecule-induced retinal neuron differentiation into a scalable 3D sphere culture system under matrix- and carrier-free conditions by, e.g., using spinner flasks, paving the path for a current general manufacture practice (cGMP)-compliant process to scale-up retinal neuron production from hPSCs for cell replacement therapy.

### Definitions

For convenience, certain terms employed in the specification, examples, and appended claims are collected here. Unless defined otherwise, all technical and scientific terms used herein have the same meaning as commonly understood by one of ordinary skill in the art to which this disclosure belongs.

The articles "a" and "an" are used herein to refer to one or to more than one (i.e., at least one) of the grammatical object of the article. By way of example, "an element" means one element or more than one element.

As used herein, the term "about" means within 20%, more preferably within 10% and most preferably within 5%. The term "substantially" means more than 50%, preferably more than 80%, and most preferably more than 90% or 95%.

As used herein, "a plurality of" means more than 1, e.g., 2, 3, 4, 5, 6, 7, 8, 9, 10, 11, 12, 13, 14, 15, 16, 17, 18, 19, 20, or more, e.g., 25, 30, 40, 50, 60, 70, 80, 90, 100, 200, 300, 400, 500, or more, or any integer there between.

As used herein the term "comprising" or "comprises" is used in reference to compositions, methods, and respective component(s) thereof, that are present in a given embodiment, yet open to the inclusion of unspecified elements.

As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

The term "embryonic stem cells" (ES cells) refers to pluripotent cells derived from the inner cell mass of blastocysts or morulae that have been serially passaged as cell lines. The ES cells may be derived from parthenogenesis etc. The term "human embryonic stem cells" (hES cells) refers to human ES cells. The generation of reference ESC is disclosed in US Patent Nos. 5,843,780; 6,200,806, and reference ESC obtained from the inner cell mass of blastocysts derived from somatic cell nuclear transfer are described in US Patent Nos. 5,945,577; 5,994,619; 6,235,970. The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Exemplary distinguishing embryonic stem cell characteristics include, without limitation, gene expression profile, proliferative capacity, differentiation capacity, karyotype, responsiveness to particular culture conditions, and the like.

The term "pluripotent" as used herein refers to a cell with the capacity, under different conditions, to differentiate to more than one differentiated cell type, and preferably to differentiate to cell types characteristic of all three germ cell layers. Pluripotent cells are characterized primarily by their ability to differentiate to more than one cell type, preferably to all three germ layers, using, for example, a nude mouse teratoma formation assay. Such cells include hES cells, and adult-derived stem cells. Pluripotent stem cells may be genetically modified or not genetically modified. Genetically modified cells may include markers such as fluorescent proteins to facilitate their identification. Pluripotency is also evidenced by the expression of embryonic stem (ES) cell markers, although the preferred test for pluripotency is the demonstration of the capacity to differentiate into cells of each of the three germ layers. It should be noted that simply culturing such cells does not, on its own, render them pluripotent. Reprogrammed pluripotent cells (e.g., iPS cells as that term is defined herein) also have the characteristic of the capacity of extended passaging without loss of growth potential, relative to primary cell parents, which generally have capacity for only a limited number of divisions in culture.

As used herein, the terms "iPS cell" and "induced pluripotent stem cell" are used interchangeably and refers to a pluripotent stem cell artificially derived (e.g., induced or by complete reversal) from a nonpluripotent cell, typically an adult somatic cell, for example, by inducing a forced expression of one or more genes.

The term "reprogramming" as used herein refers to the process that alters or reverses the differentiation state of a somatic cell, such that the developmental clock of a nucleus is reset; for example, resetting the developmental state of an adult differentiated cell nucleus so that it can carry out the genetic program of an early embryonic cell nucleus, making all the proteins required for embryonic development. Reprogramming as disclosed herein encompasses complete reversion of the differentiation state of a somatic cell to a pluripotent or totipotent cell. Reprogramming generally involves alteration, e.g., reversal, of at least some of the heritable patterns of nucleic acid modification (e.g., methylation), chromatin condensation, epigenetic changes, genomic imprinting, etc., that occur during cellular differentiation as a zygote develops into an adult.

The terms "renewal" or "self-renewal" or "proliferation" are used interchangeably herein, are used to refer to the ability of stem cells to renew themselves by dividing into the same non-specialized cell type over long periods, and/or many months to years. In some instances, proliferation refers to the expansion of cells by the repeated division of single cells into two identical daughter cells.

The term "culture" or "culturing" as used herein refers to in vitro laboratory procedures for maintaining cell viability and/or proliferation.

The term "carrier-free three-dimension sphere" culture or culturing refers to a technique of culturing the cells in nonadherent conditions such that the cells can form spheres by themselves without any carriers. A conventional method for culturing cells having adhesiveness is characterized in that cells are cultured on a plane of a vessel such as a petri dish (two-dimensional culture). In contrast, in the three-dimensional cultivation method, no adherence cue is provided to the cells and the culture is largely dependent on cell-cell contacts.

As used herein, "carriers" or "microcarriers" refer to solid spherical beads made with plastic, ceramics or other materials such as gelatin or hydrogel, designed to provide adherent surface for suspension cell culture. Carrier with other form or shape have also been reported such as fibrous structure.

The term "sphere" or "spheroid" means a three-dimensional spherical or substantially spherical cell agglomerate or aggregate. In some embodiments, extracellular matrices can be used to help the cells to move within their spheroid similar to the way cells would move in living tissue. The most common types of ECM used are basement membrane extract or collagen. In some embodiments, a matrix- or scaffold-free spheroid culture can also be used, where cells are growing suspended in media. This could be achieved either by continuous spinning or by using low-adherence plates. In embodiments, spheres can be created from single culture or co-culture techniques such as hanging drop, rotating culture, forced-floating, agitation, or concave plate methods (see, e.g., Breslin et al., Drug Discovery Today 2013, 18, 240-249; Pampaloni et al., Nat. Rev. Mol. Cell Biol. 2007, 8, 839-845; Hsiao et al., Biotechnol. Bioeng. 2012, 109, 1293-1304; and Castaneda et al., J. Cancer Res. Clin. Oncol. 2000, 126, 305-310). In some embodiments, the size of the spheres can grow during 3D culturing.

The term "culture medium" is used interchangeably with "medium" and refers to any medium that allows cell proliferation. The suitable medium need not promote maximum proliferation, only measurable cell proliferation. In some embodiments, the culture medium maintains the cells in a pluripotent state. In some embodiments, the culture medium encourages the cells (e.g., pluripotent cells) to differentiate into, e.g., eye early and late committed retinal neural progenitors (CRNP) and photoreceptor precursor cells (PRPC). A few exemplary basal media used herein include DMEM/F-12 (Dulbecco's Modified Eagle Medium/Nutrient Mixture F-12; available from Thermo Fisher Scientific), Growth Factor-Free NutriStem^{®} Medium which contains no bFGF or TGFb (GF-free NutriStem^{®}, used interchangeably with Pluriton^{™} ("PL"), available from Biological Industries) and Neurobasal^{™} medium (available from Thermo Fisher Scientific). Each can be supplemented with one or more of: suitable buffer (e.g., HEPES (4-(2-hydroxyethyl)-1-piperazineethanesulfonic acid)), chemically-defined supplements such as N2 (0.1-10%, e.g., 1%) and B27 (0.1-10%, e.g., 1%) serum-free supplements (available from Thermo Fisher Scientific), antibiotics such as penicillin/streptomycin (0.1-10%, e.g., 1%), MEM non-essential amino acids (Eagle's minimum essential medium (MEM) which is composed of balanced salt solutions, amino acids and vitamins that are essential for the growth of cultured cells, which, when supplemented with non-essential amino acids, makes MEM non-essential amino acid solution), glucose (0.1-10%, e.g., 0.30%), L-glutamine (e.g., GlutaMAX^{™}), human brain-derived neurotrophic factor (BDNF), ascorbic acid, and/or DAPT (N-[N-(3,5-difluorophenacetyl)-1-alanyl]-S-phenylglycine t-butyl ester). Factors for inducing differentiation such as Sonic Hedgehog, Heparin, IWR-1e, SB431542, LDN193189 and IGF1 as disclosed herein can also be added to the medium.

The term "differentiated cell" as used herein refers to any cell in the process of differentiating into a somatic cell lineage or having terminally differentiated. For example, embryonic cells can differentiate into an epithelial cell lining the intestine. Differentiated cells can be isolated from a fetus or a live born animal, for example.

In the context of cell ontogeny, the adjective "differentiated", or "differentiating" is a relative term meaning a "differentiated cell" is a cell that has progressed further down the developmental pathway than the cell it is being compared with. Thus, stem cells can differentiate to lineage-restricted precursor cells (such as a mesodermal stem cell), which in turn can differentiate into other types of precursor cells further down the pathway (such as a photoreceptor precursor), and then to an end-stage differentiated cell, which plays a characteristic role in a certain tissue type, and may or may not retain the capacity to proliferate further.

The terms "enriching" or "enriched" are used interchangeably herein and mean that the yield (fraction) of cells of one type is increased by at least 10% over the fraction of cells of that type in the starting culture or preparation.

The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and nonproteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In certain embodiments, agents are small molecule having a chemical moiety. For example, chemical moieties included unsubstituted or substituted alkyl, aromatic, or heterocyclyl moieties including macrolides, leptomycins and related natural products or analogues thereof. Compounds can be known to have a desired activity and/or property, or can be selected from a library of diverse compounds.

The term "small molecule" refers to an organic compound having multiple carbon-carbon bonds and a molecular weight of less than 1500 daltons. Typically such compounds comprise one or more functional groups that mediate structural interactions with proteins, e.g., hydrogen bonding, and typically include at least an amine, carbonyl, hydroxyl or carboxyl group, and in some embodiments at least two of the functional chemical groups. The small molecule agents may comprise cyclic carbon or heterocyclic structures and/or aromatic or polyaromatic structures substituted with one or more chemical functional groups and/or heteroatoms.

The term "marker" as used herein is used to describe the characteristics and/or phenotype of a cell. Markers can be used for selection of cells comprising characteristics of interests. Markers will vary with specific cells. Markers are characteristics, whether morphological, functional or biochemical (enzymatic) characteristics of the cell of a particular cell type, or molecules expressed by the cell type. Preferably, such markers are proteins, and more preferably, possess an epitope for antibodies or other binding molecules available in the art. However, a marker may consist of any molecule found in a cell including, but not limited to, proteins (peptides and polypeptides), lipids, polysaccharides, nucleic acids and steroids. Examples of morphological characteristics or traits include, but are not limited to, shape, size, and nuclear to cytoplasmic ratio. Examples of functional characteristics or traits include, but are not limited to, the ability to adhere to particular substrates, ability to incorporate or exclude particular dyes, ability to migrate under particular conditions, and the ability to differentiate along particular lineages. Markers may be detected by any method available to one of skill in the art. Markers can also be the absence of a morphological characteristic or absence of proteins, lipids etc. Markers can be a combination of a panel of unique characteristics of the presence and absence of polypeptides and other morphological characteristics.

The term "isolated population" with respect to an isolated population of cells as used herein refers to a population of cells that has been removed and separated from a mixed or heterogeneous population of cells. In some embodiments, an isolated population is a substantially pure population of cells as compared to the heterogeneous population from which the cells were isolated or enriched from.

The term "substantially pure", with respect to a particular cell population, refers to a population of cells that is at least about 75%, preferably at least about 85%, more preferably at least about 90%, and most preferably at least about 95% pure, with respect to the cells making up a total cell population. Recast, the terms "substantially pure" or "essentially purified", with regard to a population of definitive endoderm cells, refers to a population of cells that contain fewer than about 20%, more preferably fewer than about 15%, 10%, 8%, 7%, most preferably fewer than about 5%, 4%, 3%, 2%, 1%, or less than 1%, of cells that are not definitive endoderm cells or their progeny as defined by the terms herein. In some embodiments, the present disclosure encompasses methods to expand a population of definitive endoderm cells, wherein the expanded population of definitive endoderm cells is a substantially pure population of definitive endoderm cells. Similarly, with regard to a "substantially pure" or "essentially purified" population of pluripotent stem cells, refers to a population of cells that contain fewer than about 20%, more preferably fewer than about 15%, 10%, 8%, 7%, most preferably fewer than about 5%, 4%, 3%, 2%, 1%.

"Retina" refers to the neural cells of the eye, which are layered into three nuclear layers comprised of photoreceptors, horizontal cells, bipolar cells, amacrine cells, Müller glial cells and ganglion cells.

"Progenitor cell" refers to a cell that remains mitotic and can produce more progenitor cells or precursor cells or can differentiate to an end fate cell lineage.

"Precursor cell" refers to a cell capable of differentiating to an end fate cell lineage.

In embodiments of the disclosure, a "retinal neural progenitor cell" refers to a cell differentiated from embryonic stem cells or induced pluripotent stem cells, that expresses the cell markers PAX6 and CHX10. This can include early and late committed retinal neuron progenitors, which can express the markers PAX6, RAX1 and CHX10.

In embodiments of the disclosure, a "photoreceptor precursor cell" (PRPC) refers to cells differentiated from embryonic stem cells or induced pluripotent stem cells and that expresses the marker PAX6 while not expressing the marker CHX10 (i.e., CHX10-). These cells transiently express CHX10 at retinal neural progenitor stage, but the CHX10 expression is turned off when cells differentiate into the photoreceptor progenitor stage. PRPCs can also express the markers CRX, NRL, NR2E3, and ThRB2.

Also, "photoreceptor" may refer to post-mitotic cells differentiated from embryonic stem cells or induced pluripotent stem cells and that expresses the cell marker rhodopsin (RHOD) or any of the three cone opsins (e.g., M-OPSIN), and optionally express the rod or cone cGMP phosphodiesterase. The photoreceptors may also express the marker recoverin (REC), which is found in photoreceptors. The photoreceptors may be rod and/or cone photoreceptors.

"Photoreceptors-like cell" is a cell expressing most or all of photoreceptor-specific markers, but have not been tested for its function.

The term "treatment" or "treating" means administration of a substance for purposes including: (i) preventing the disease or condition, that is, causing the clinical symptoms of the disease or condition not to develop; (ii) inhibiting the disease or condition, that is, arresting the development of clinical symptoms; and/or (iii) relieving the disease or condition, that is, causing the regression of clinical symptoms.

Various aspects of the disclosure are described in further detail below. Additional definitions are set out throughout the specification.

### Pluripotent Stem Cells

In various embodiments, PRPCs and photoreceptor cells can be produced from human pluripotent stem cells (hPSCs), including but not limited to human embryonic stem cells (hESCs), human parthenogenetic stem cells (hpSCs), nuclear transfer derived non-human stem cells, and induced pluripotent stem cells (iPSCs). Methods of obtaining such hPSCs are well known in the art.

Pluripotent stem cells are defined functionally as stem cells that are: (a) capable of inducing teratomas when transplanted in immunodeficient (SCID) mice; (b) capable of differentiating to cell types of all three germ layers (e.g., can differentiate to ectodermal, mesodermal, and endodermal cell types); and (c) express one or more markers of embryonic stem cells (e.g., express OCT4, alkaline phosphatase. SSEA-3 surface antigen, SSEA-4 surface antigen, NANOG, TRA-1-60, TRA-1-81, SOX2, REX1, etc). In certain embodiments, pluripotent stem cells express one or more markers selected from the group consisting of: OCT4, alkaline phosphatase, SSEA-3, SSEA-4, TRA-1-60, and TRA-1-81. Exemplary pluripotent stem cells can be generated using, for example, methods known in the art.. Further exemplary pluripotent stem cells include induced pluripotent stem cells (iPSCs) generated by reprogramming a somatic cell by expressing a combination of factors (herein referred to as reprogramming factors). The iPSCs can be generated using fetal, postnatal, newborn, juvenile, or adult somatic cells.

In certain embodiments, factors that can be used to reprogram somatic cells to pluripotent stem cells include, for example, a combination of OCT4 (sometimes referred to as OCT3/4), SOX2, c-Myc, and Klf4. In other embodiments, factors that can be used to reprogram somatic cells to pluripotent stem cells include, for example, a combination of OCT4, SOX2, NANOG, and LIN28. In certain embodiments, at least two reprogramming factors are expressed in a somatic cell to successfully reprogram the somatic cell. In other embodiments, at least three reprogramming factors are expressed in a somatic cell to successfully reprogram the somatic cell. In other embodiments, at least four reprogramming factors are expressed in a somatic cell to successfully reprogram the somatic cell. In other embodiments, additional reprogramming factors are identified and used alone or in combination with one or more known reprogramming factors to reprogram a somatic cell to a pluripotent stem cell. Induced pluripotent stem cells are defined functionally and include cells that are reprogrammed using any of a variety of methods (integrative vectors, non-integrative vectors, chemical means, etc). Pluripotent stem cells may be genetically modified or otherwise modified to increase longevity, potency, homing, to prevent or reduce alloimmune responses or to deliver a desired factor in cells that are differentiated from such pluripotent cells (for example, photoreceptors, photoreceptor progenitor cells, rods, cones, etc. and other cell types described herein, e.g., in the examples).

Induced pluripotent stem cells (iPS cells or iPSC) can be produced by protein transduction of reprogramming factors in a somatic cell. In certain embodiments, at least two reprogramming proteins are transduced into a somatic cell to successfully reprogram the somatic cell. In other embodiments, at least three reprogramming proteins are transduced into a somatic cell to successfully reprogram the somatic cell. In other embodiments, at least four reprogramming proteins are transduced into a somatic cell to successfully reprogram the somatic cell.

The pluripotent stem cells can be from any species. Embryonic stem cells have been successfully derived in, for example, mice, multiple species of non-human primates, and humans, and embryonic stem-like cells have been generated from numerous additional species. Thus, one of skill in the art can generate embryonic stem cells and embryo-derived stem cells from any species, including but not limited to, human, non-human primates, rodents (mice, rats), ungulates (cows, sheep, etc), dogs (domestic and wild dogs), cats (domestic and wild cats such as lions, tigers, cheetahs), rabbits, hamsters, gerbils, squirrel, guinea pig, goats, elephants, panda (including giant panda), pigs, raccoon, horse, zebra, marine mammals (dolphin, whales, etc.) and the like. In certain embodiments, the species is an endangered species. In certain embodiments, the species is a currently extinct species.

Similarly, iPS cells can be from any species. These iPS cells have been successfully generated using mouse and human cells. Furthermore, iPS cells have been successfully generated using embryonic, fetal, newborn, and adult tissue. Accordingly, one can readily generate iPS cells using a donor cell from any species. Thus, one can generate iPS cells from any species, including but not limited to, human, non-human primates, rodents (mice, rats), ungulates (cows, sheep, etc), dogs (domestic and wild dogs), cats (domestic and wild cats such as lions, tigers, cheetahs), rabbits, hamsters, goats, elephants, panda (including giant panda), pigs, raccoon, horse, zebra, marine mammals (dolphin, whales, etc.) and the like. In certain embodiments, the species is an endangered species. In certain embodiments, the species is a currently extinct species.

Induced pluripotent stem cells can be generated using, as a starting point, virtually any somatic cell of any developmental stage. For example, the cell can be from an embryo, fetus, neonate, juvenile, or adult donor. Exemplary somatic cells that can be used include fibroblasts, such as dermal fibroblasts obtained by a skin sample or biopsy, synoviocytes from synovial tissue, foreskin cells, cheek cells, or lung fibroblasts. Although skin and cheek provide a readily available and easily attainable source of appropriate cells, virtually any cell can be used. In certain embodiments, the somatic cell is not a fibroblast.

The induced pluripotent stem cell may be produced by expressing or inducing the expression of one or more reprogramming factors in a somatic cell. The somatic cell may be a fibroblast, such as a dermal fibroblast, synovial fibroblast, or lung fibroblast, or a non-fibroblastic somatic cell. The somatic cell may be reprogrammed through causing expression of (such as through viral transduction, integrating or non-integrating vectors, etc.) and/or contact with (e.g., using protein transduction domains, electroporation, microinjection, cationic amphiphiles, fusion with lipid bilayers containing, detergent permeabilization, etc.) at least 1, 2, 3, 4, 5 reprogramming factors. The reprogramming factors may be selected from OCT3/4, SOX2, NANOG, LIN28, C-MYC, and KLF4. Expression of the reprogramming factors may be induced by contacting the somatic cells with at least one agent, such as a small organic molecule agents, that induce expression of reprogramming factors.

Further exemplary pluripotent stem cells include induced pluripotent stem cells generated by reprogramming a somatic cell by expressing or inducing expression of a combination of factors ("reprogramming factors"). iPS cells may be obtained from a cell bank. The making of iPS cells may be an initial step in the production of differentiated cells. iPS cells may be specifically generated using material from a particular patient or matched donor with the goal of generating tissue-matched PHRPS or photoreceptor cells. iPSCs can be produced from cells that are not substantially immunogenic in an intended recipient, e.g., produced from autologous cells or from cells histocompatible to an intended recipient.

The somatic cell may also be reprogrammed using a combinatorial approach wherein the reprogramming factor is expressed (e.g., using a viral vector, plasmid, and the like) and the expression of the reprogramming factor is induced (e.g., using a small organic molecule.) For example, reprogramming factors may be expressed in the somatic cell by infection using a viral vector, such as a retroviral vector or a lentiviral vector. Also, reprogramming factors may be expressed in the somatic cell using a non-integrative vector, such as an episomal plasmid. See, e.g., Yu et al., Science. 2009 May 8; 324(5928):797-801. When reprogramming factors are expressed using non-integrative vectors, the factors may be expressed in the cells using electroporation, transfection, or transformation of the somatic cells with the vectors. For example, in mouse cells, expression of four factors (OCT3/4, SOX2, C-MYC, and KLF4) using integrative viral vectors is sufficient to reprogram a somatic cell. In human cells, expression of four factors (OCT3/4, SOX2, NANOG, and LIN28) using integrative viral vectors is sufficient to reprogram a somatic cell.

Once the reprogramming factors are expressed in the cells, the cells may be cultured. Over time, cells with ES characteristics appear in the culture dish. The cells may be chosen and subcultured based on, for example, ES morphology, or based on expression of a selectable or detectable marker. The cells may be cultured to produce a culture of cells that resemble ES cells-these are putative iPS cells.

To confirm the pluripotency of the iPS cells, the cells may be tested in one or more assays of pluripotency. For example, the cells may be tested for expression of ES cell markers; the cells may be evaluated for ability to produce teratomas when transplanted into SCID mice; the cells may be evaluated for ability to differentiate to produce cell types of all three germ layers. Once a pluripotent iPSC is obtained it may be used to produce cell types disclosed herein, e.g., photoreceptor progenitor cells, photoreceptor cells, rods, cones, etc. and other cell types described herein.

Another method of obtaining hPSCs is by parthenogenesis. "Parthenogenesis" ("parthenogenically activated" and "parthenogenetically activated" is used herein interchangeably) refers to the process by which activation of the oocyte occurs in the absence of sperm penetration, and refers to the development of an early stage embryo comprising trophectoderm and inner cell mass that is obtained by activation of an oocyte or embryonic cell, e.g., blastomere, comprising DNA of all female origin. In a related aspect, a "parthenote" refers to the resulting cell obtained by such activation. In another related aspect, "blastocyst: refers to a cleavage stage of a fertilized of activated oocyte comprising a hollow ball of cells made of outer trophoblast cells and an inner cell mass (ICM). In a further related aspect, "blastocyst formation" refers to the process, after oocyte fertilization or activation, where the oocyte is subsequently cultured in media for a time to enable it to develop into a hollow ball of cells made of outer trophoblast cells and ICM (e.g., 5 to 6 days).

Another method, not part of the invention, of obtaining hPSCs is through nuclear transfer. As used herein, "nuclear transfer" refers to the fusion or transplantation of a donor cell or DNA from a donor cell into a suitable recipient cell, typically an oocyte of the same or different species that is treated before, concomitant or after transplant or fusion to remove or inactivate its endogenous nuclear DNA. The donor cell used for nuclear transfer include embryonic and differentiated cells, e.g., somatic and germ cells. The donor cell may be in a proliferative cell cycle (Gl, G2, S or M) or non-proliferating (GO or quiescent). Preferably, the donor cell or DNA from the donor cell is derived from a proliferating mammalian cell culture, e.g., a fibroblast cell culture. The donor cell optionally may be transgenic, i.e., it may comprise one or more genetic addition, substitution or deletion modifications.

A further method for obtaining hPSCs is through the reprogramming of cells to obtain induced pluripotent stem cells. Takahashi et al. (Cell 131, 861-872 (2007)) have disclosed methods for reprogramming differentiated cells, without the use of any embryo or ES (embryonic stem) cell, and establishing an inducible pluripotent stem cell having similar pluripotency and growing abilities to those of an ES cell. Nuclear reprogramming factors for differentiated fibroblasts include products of the following four genes: an Oct family gene; a Sox family gene; a Klf family gene; and a Myc family gene.

The pluripotent state of the cells is preferably maintained by culturing cells under appropriate conditions, for example, by culturing on a fibroblast feeder layer or another feeder layer or culture that includes leukemia inhibitory factor (LIF). The pluripotent state of such cultured cells can be confirmed by various methods, e.g., (i) confirming the expression of markers characteristic of pluripotent cells; (ii) production of chimeric animals that contain cells that express the genotype of the pluripotent cells; (iii) injection of cells into animals, e.g., SCID mice, with the production of different differentiated cell types in vivo; and (iv) observation of the differentiation of the cells (e.g., when cultured in the absence of feeder layer or LIF) into embryoid bodies and other differentiated cell types in vitro.

The pluripotent state of the cells used in the present disclosure can be confirmed by various methods. For example, the cells can be tested for the presence or absence of characteristic ES cell markers. In the case of human ES cells, examples of such markers are identified supra, and include SSEA-4, SSEA-3, TRA-1-60, TRA-1-81 and OCT 4, and are known in the art.

Also, pluripotency can be confirmed by injecting the cells into a suitable animal, e.g., a SCID mouse, and observing the production of differentiated cells and tissues. Still another method of confirming pluripotency is using the subject pluripotent cells to generate chimeric animals and observing the contribution of the introduced cells to different cell types.

Yet another method of confirming pluripotency is to observe ES cell differentiation into embryoid bodies and other differentiated cell types when cultured under conditions that favor differentiation (e.g., removal of fibroblast feeder layers). This method has been utilized and it has been confirmed that the subject pluripotent cells give rise to embryoid bodies and different differentiated cell types in tissue culture.

hPSCs can be maintained in culture in a pluripotent state by routine passage until it is desired that neural stem cells be derived.

### 3D Matrix- and Carrier-free Sphere Culture to Produce Photoreceptors

For a practical application of hPSCs in cell therapy, further refinement to large-scales and 3D culture systems are necessary. Various 3D sphere culture procedures can be used, such as include forced-floating methods that modify cell culture surfaces and thereby promote 3D culture formation by preventing cells from attaching to their surface; the hanging drop method which supports cellular growth in suspension; and agitation/rotary systems that encourage cells to adhere to each other to form 3D spheroids.

One method for generating 3D spheroids is to prevent their attachment to the vessel surface by modifying the surface, resulting in forced-floating of cells. This promotes cell-cell contacts which, in turn, promotes multi-cellular sphere formation. Exemplary surface modification includes poly-2-hydroxyethyl methacrylate (poly-HEMA) and agarose.

The hanging drop method of 3D spheroid production uses a small aliquot (typically 20 ml) of a single cell suspension which is pipetted into the wells of a tray. Similarly to forced-floating, the cell density of the seeding suspension (e.g. 50, 100, 500 cells/well, among others) can be altered as relevant, depending on the required size of spheroids. Following cell seeding, the tray is subsequently inverted and aliquots of cell suspension turn into hanging drops that are kept in place due to surface tension. Cells accumulate at the tip of the drop, at the liquid-air interface, and are allowed to proliferate.

Agitation-based approaches for the production of 3D spheroids can be loosely placed into two categories as (i) spinner flask bioreactors and (ii) rotational culture systems. The general principle behind these methods is that a cell suspension is placed into a container and the suspension is kept in motion, that is, either it is gently stirred or the container is rotated. The continuous motion of the suspended cells means that cells do not adhere to the container walls, but instead form cell-cell interactions. Spinner flask bioreactors (typically known as "spinners") include a container to hold the cell suspension and a stirring element to ensure that the cell suspension is continuously mixed. Rotating cell culture bioreactors function by similar means as the spinner flask bioreactor but, instead of using a stirring bar/rod to keep cell suspensions moving, the culture container itself is rotated.

In some embodiments, provided herein is a spinner flask based 3D sphere culture protocol. A plurality of hPSCs can be continuously cultured as substantially uniform spheres in spinner flasks with a defined culture medium in the absence of feeder cells and matrix. The culture medium can be any defined, xeno-free, serum-free cell culture medium designed to support the growth and expansion of hPSCs such as hiPSC and hES. In one example, the medium is NutriStem^{®} medium. In some embodiments, the medium can be mTeSR1, mTeSR2, or E8 medium, or other stem cell medium. The medium can be supplemented with small molecule inhibitor of Rho-associated, coiled-coil containing protein kinase (ROCK) such as Y27632 or other ROCK inhibitors such as Thiazovivin, ROCK II inhibitor (e.g., SR3677) and GSK429286A. With this suspension culture system, hPSC cultures can be serially passaged and consistently expanded for at least 10 passages. A typical passaging interval for 3D-hiPSC sphere can be about 3-6 days, at which time spheres can grow into a size of about 230-260 µm in diameter. Sphere size can be monitored by taking an aliquot of the culture and observing using, e.g., microscopy. Then the spheres can be dissociated into single (or substantially single) cells using, e.g., an enzyme with proteolytic and collagenolytic activity for the detachment of primary and stem cell lines and tissues. In one example, the enzyme is Accutase^{®}, or TrypLE, or Trypsin/EDTA. Thereafter, the disassociated cells can be reaggregated to reform spheres in spinner flasks under continuous agitation at, e.g., 60-70RPM. Spheres gradually increased in size while maintaining a uniform structure together with a high pluripotency marker expression (OCT4) and a normal karyotype after at least 3-5 repeated passages. As used herein, a "passage" is understood to mean a cell sphere culture grown from single cells into spheres of a desirable size, at which time the spheres are disassociated into single cells and seeded again for the next passage. A passage can take about 3-6 days for 3D-hiPSC spheres, or longer or shorter, depending on the type of hPSCs and culturing conditions. Once sufficient amounts of 3D-hPSC spheres are obtained, they can be subject to 3D sphere retinal neuron differentiation, as described in more detail below.

To generate retinal neuronal progenitors at different developmental stages from hPSCs, 3D-hPSC spheres in suspension can be directly induced in a stepwise fashion with mainly small molecules **(****Figure 2****).** In some embodiments, this can be done in 3D spinner flasks, or other 3D sphere culturing methods. In various embodiments, continuous 3D sphere culture can be integrated with several dissociation/reaggregation steps, while small molecules can be added at different developmental stages to induce retinal neuron differentiation. Instead of using protein factors for induction of hPSC differentiation toward retinal lineages as previously reported, small molecules are used where possible, the quality of which can be easily controlled, to sequentially differentiate hPSC spheres to different developmental stages of retinal cells.

As shown in Figure 2, hPSCs (e.g., hiPSCs) can first be seeded as single cells (e.g., 1 x 10⁶ cells / mL) in a defined medium (e.g., NutriStem^{®} ("NS") medium) supplemented with ROCK inhibitor (e.g., Y27632, "Y") in spinner flasks to form spheres. 24 hours later, designated as D0 of induction, hiPSC spheres can be first treated with dual SMAD inhibitors SB431542 ("SB") and LDN193189 ("LDN") to block the signal transduction of activin/transforming growth factor β (TGF-β) and bone morphogenetic protein (BMP) and to facilitate neural patterning, then Wnt inhibitor IWR-1e, IGF1 (an inducer of eye filed cell development) and heparin can be added to further induce retinal neural lineage commitment.

For PRPC differentiation, a gradual adaptation to NIM-3D medium can be achieved through a dilution series of Pluriton^{™}/GF-free NutriStem^{®} and NIM-3D with the inducing factors mentioned above. For example, gradual adaption from 100% Pluriton^{™}/GF-free NutriStem^{®} to 100% NIM-3D can include intermediate culturing with Pluriton^{™}/GF-free NutriStem^{®} and NIM-3D sequentially at 75%:25%, 50%:50%, and 25%:75%, with the cells spending 2-6 days in each medium composition. Other dilution series can also be used. NIM-3D (Neural Induction Medium-3D) (used interchangeably with "NIM") basal medium contains DMEM/F12 with HEPES, N2 (0.1-10%, e.g., 1%) and B27 (0.1-10%, e.g., 1%) serum-free supplements, penicillin/streptomycin (0.1-10%, e.g., 1%), MEM non-essential amino acids, and glucose (0.1-10%, e.g., 0.30%). Heparin can be withdrawn during neural commitment and IWR-1e upon complete adaptation in NIM-3D medium.

Thereafter, spheres can be adapted to PRPC-3D photoreceptor differentiation medium through a 50/50 adaptation containing NIM-3D/PRPC-3D medium. PRPC-3D medium (also referred to as "PRPM" in, e.g., Figure 11) contains Neurobasal^{™} medium, N2 (0.1-10%, e.g., 1%) and B27 (0.1-10%, e.g., 1%) serum-free supplements, penicillin/streptomycin (0.1-10%, e.g., 1%), MEM non-essential amino acids, and glucose (0.1-10%, e.g., 0.30%). SB431542, LDN193189 and IGF1 can be withdrawn upon initiation of adaptation into NIM-3D/PRPC-3D medium.

Another exemplary differentiation protocol is illustrated in Figure 11. The key difference from the protocol shown in Figure 2 is the use of Sonic Hedgehog (SHH) in place of heparin. Surprisingly, SHH is a more effective alternative to other mitogen-activated proteins such as heparin in achieving neuronal induction and the proliferation of retinal progenitors, as well as maintaining neurogenesis of PAX6-positive cells.

During differentiation, spheres can be dissociated into single cells using a cell-dissociation enzyme such as TrypLE (Thermo Fisher Scientific), Accutase, or Trypsin/EDTA at different time points. PRPCs can be generated by continuous dissociation of spheres into single cells and reaggregation into spinner flasks every 2-5 weeks, when sphere diameter typically or on average reaches about 300-500 µm or about 350-450 µm to avoid generating hypoxic cells in the center of the spheres. It should be noted that sphere size over about 450 or 500 µm in diameter can be undesirable as this may prevent oxygen, nutrients and differentiation inducing factors/molecules from penetrating into the central core of the sphere, thereby resulting in necrosis and other delicious causes leading to cell death at the core. As such, once the spheres grow into an average size of about 300-400 µm or about 350-450 µm in diameter, the spheres can be dissociated into single (or substantially single) cells using various enzymes for cell disassociation known in the art.

Without wishing to be bound by theory, it is believed that if the spheres grow too big (e.g., over 500 or over 400 µm in diameter), then the cells close to the center of the spheres may risk malnutrition. Thus, it can be desirable to control sphere size in some embodiments. In certain embodiments, the spheres can be dissociated when they reach about 300-500 µm, about 350-450 µm, or about 230-260 µm in diameter.

Once disassociated, the single cell suspension can be filtered through a filter (e.g., about 10-200 or about 20-100 or about 40 µm in mesh size). Single cells can then be seeded into a spinner flask in the appropriate culture medium. Morphology of the resulting spheres (size, appearance, and ability to incorporate into spheres) can be monitored 2-3 days after each reaggregation and every week after that until next dissociation/reaggregation step. Monitoring can be done by taking an aliquot of the culture and observing using, e.g., microscopy.

As such, disclosed herein is a stepwise hPSC sphere differentiation process toward different developmental stages of retinal neurons, including early and late committed retinal neuronal progenitors (CRNP), photoreceptor precursor cells (PRPC) and photoreceptor-like cells. Compared to previous reported differentiation protocols, the 3D sphere platform possesses the following advantages:
1) **Defined culture medium with small molecules, but without undefined matrix.** In the 3D sphere culture systems disclosed herein, from hPSC maintenance and expansion to 3D sphere retinal differentiation, no serum and undefined matrix or carrier is added to the culture medium. Furthermore, small molecules were used to replace protein factors, for which the quality and consistence can be easily controlled, making the 3D sphere system more consistence and repeatable than previously reported systems;
2) **A robust platform for cell process and manufacture.** The transition from a 3D hPSC sphere expansion culture to a 3D sphere differentiation process is straight forward, no cell manipulation is needed such as cell attachment to surface or matrix embedment, only culture medium replacement is required, which makes the transition smoothness; in addition, retinal neurons at different developmental stages were generated during the process, and these cells can be cryopreserved for further differentiation at a later time, rendering the quality control process much easier;
3) **Uniformity and integrity of 3D spheres leading to synchronized retinal neuron differentiation.** By controlling the speed of stirring/agitation and initial cell density, the diameters and integrity of 3D spheres in hPSC expansion and induced differentiation process can be well controlled for a long time, typically 3 to 4 months. This allows oxygen, nutrients and differentiation inducing factors/molecules to penetrate into the central core of sphere, avoiding necrosis and other delicious causes leading to cell death, which is common in no-controlled embryoid body and organoid systems. Keeping the uniformity and integrity of the 3D spheres results in a synchronized differentiation process with the generation of pure retinal neuron population;
4) **Scalable process for large quantity production of desirable cells.** While the Examples used a 30-50 ml bioreactor for the proof-of-concept study, this can be easily extended to multiple ones and large volume bioreactors. Each 30-50 ml bioreactor usually generates about 3 X 10⁸ retinal neuron cells, equivalent to the capacity of 20-25 T-75 tissue culture flasks, which makes this system a cost-effective and easy manageable process for cell production;
5) **Generation of more homogenous cell population.** During the process of induced differentiation, the dissociation/reaggregation steps are integrated for sphere reformation when splitting these cells, which serves as a purification step to eliminate non-neuron cells and enrich neuron cells. These steps result in the generation of different developmental stage retinal cells at different steps including early and late CRNPs, PRPCs and photoreceptor-like cells with high purity (about 95%) without contamination of undifferentiated pluripotent stem cells; and
6) **An adaptable system that can be extend for the generation of other neuron types.** As this 3D sphere differentiation process is a multistep procedure and each step generates unique neuronal processors, by modifying differentiation induction conditions, this process can be easily extended to generate other neuronal cell types, including but not limited to other retinal neuron cells such as retinal ganglion cells and progenitors, bipolar cells, muller cells, horizontal cells and amacrine cells, and other general neuron cells.

In various embodiments, provided herein is a new, efficient and defined 3D sphere platform to generate desirable cells from hPSCs, specifically PRPCs and photoreceptor-like cells that can be used for photoreceptor replacement therapy in blind patients. This system is not only amenable to large-scale production efforts, but also eliminated dependence on animal serum and matrix, plus supplement of small molecules instead of protein factors, thus rendering it friendly to cGMP compliant cell manufacturing protocol and making the process more amenable to clinical translation.

### Photoreceptor Replacement Therapy

Retinal diseases often result in blindness due to loss of post-mitotic neuronal cells. Among the retinal diseases are rod or cone dystrophies, retinal degeneration, retinitis pigmentosa, diabetic retinopathy, macular degeneration, Leber congenital amaurosis and Stargardt disease. In most retinal degenerations, cell loss is primarily in the outer nuclear layer which includes rod and cone photoreceptors. With the loss of post-mitotic neuronal cell populations, an exogenous source of new cells as a replacement for photoreceptor cells is needed.

Retinal degeneration is an irreversible process that ultimately leads to blindness. Rod and cone photoreceptors in the retina are the major light sensing cells, but these cells lack the capacity to regenerate. At present, there is no treatment to regenerate lost photoreceptors, cell replacement is the only therapeutic strategy for the treatment of patients with photoreceptor loss. MacLaren et al [46] was the first group to demonstrate that transplantation of mouse post-mitotic photoreceptor precursor cells into completely blind mice restored some visual functions. These transplanted cells integrated into the ONL layer, differentiated into rod photoreceptors, formed synaptic connections and improved visual function in these animals. Recently there are several groups reported improvement of visual function in animal models with a varied range of retinal dysfunctions following transplantation of post-mitotic photoreceptor precursor cells derived from both mouse and human PSCs [9, 42, 43]. These results provide the proof-of-concept animal studies that cell replacement therapy for photoreceptor degeneration patients may work if appropriate cells are transplanted into the right place with the suitable microenvironments. Several clinical trials using hESC and hiPSC derived retinal pigment epithelium (RPE) to prevent vision loss for patients with AMD and Stargardt disease are currently undergoing [3-5], but cell transplantation for replacement of lost photoreceptors has not started yet, therefore there is a critical need for human photoreceptor precursor cells at proper developmental stages from a renewable source for cell replacement therapy. Obviously post mitotic human photoreceptor precursors from human donors do not represent a suitable source for cell replacement, differentiation of human PSCs *in vitro* to generate retinal neurons, especially postmitotic photoreceptor progenitor cells, will serve the purpose.

Accordingly, the PRPCs or photoreceptor cells disclosed herein may be formulated with a pharmaceutically acceptable carrier and used in photoreceptor replacement therapy. For example, PRPCs or photoreceptor cells may be administered alone or as a component of a pharmaceutical formulation. The subject compounds may be formulated for administration in any convenient way for use in medicine. Pharmaceutical preparations suitable for administration may comprise the PRPCs or photoreceptor cells, in combination with one or more pharmaceutically acceptable sterile isotonic aqueous or nonaqueous solutions (e.g., balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents. Exemplary pharmaceutical preparations comprises the PRPCs or photoreceptor cells in combination with ALCON^{®} BSS PLUS^{®} (a balanced salt solution containing, in each mL, sodium chloride 7.14 mg, potassium chloride 0.38 mg, calcium chloride dihydrate 0.154 mg, magnesium chloride hexahydrate 0.2 mg, dibasic sodium phosphate 0.42 mg, sodium bicarbonate 2.1 mg, dextrose 0.92 mg, glutathione disulfide (oxidized glutathione) 0.184 mg, hydrochloric acid and/or sodium hydroxide (to adjust pH to approximately 7.4) in water).

When administered, the pharmaceutical preparations for use in this disclosure may be in a pyrogen-free, physiologically acceptable form.

The preparation comprising PRPCs or photoreceptor cells used in the methods described herein may be transplanted in a suspension, gel, colloid, slurry, or mixture. Further, the preparation may desirably be encapsulated or injected in a viscous form into the vitreous humor for delivery to the site of retinal or choroidal damage. Also, at the time of injection, cryopreserved PRPCs or photoreceptor cells may be resuspended with commercially available balanced salt solution to achieve the desired osmolality and concentration for administration by subretinal injection. The preparation may be administered to an area of the pericentral macula that was not completely lost to disease, which may promote attachment and/or survival of the administered cells.

The PRPCs or photoreceptor cells of the disclosure may be delivered in a pharmaceutically acceptable ophthalmic formulation by intraocular injection. When administering the formulation by intravitreal injection, for example, the solution may be concentrated so that minimized volumes may be delivered. Concentrations for injections may be at any amount that is effective and non-toxic, depending upon the factors described herein. The pharmaceutical preparations of PRPCs or photoreceptor cells for treatment of a patient may be formulated at doses of at least about 10⁴ cells/mL. The PRPCs or photoreceptor cell preparations for treatment of a patient are formulated at doses of at least about 10³, 10⁴, 10⁵, 10⁶, 107, 10⁸, 10⁹, or 10¹⁰ PRPCs or photoreceptor cells/mL. For example, the PRPCs or photoreceptor cells may be formulated in a pharmaceutically acceptable carrier or excipient.

The pharmaceutical preparations of PRPCs or photoreceptor cells described herein may comprise at least about 1,000; 2,000; 3,000; 4,000; 5,000; 6,000; 7,000; 8,000; or 9,000 PRPCs or photoreceptor cells. The pharmaceutical preparations of PRPCs or photoreceptor cells may comprise at least about 1×10⁴, 2×10⁴, 3×10⁴, 4×10⁴, 5×10⁴, 6×10⁴, 7×10⁴, 8×10⁴, 9×10⁴, 1×10⁵, 2×10⁵, 3×10⁵, 4×10⁵, 5×10⁵, 6×10⁵, 7×10⁵, 8×10⁵, 9×10⁵, 1×10⁶, 2×10⁶, 3×10⁶, 4×10⁶, 5×10⁶, 6×10⁶, 7×10⁶, 8×10⁶, 9×10⁶, 1×10⁷, 2×10⁷, 3×10⁷, 4×10⁷, 5×10⁷, 6×10⁷, 7×10⁷, 8×10⁷, 9×10⁷, 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, or 9×10¹⁰ PRPCs or photoreceptor cells, or more or less. The pharmaceutical preparations of PRPCs or photoreceptor cells may comprise at least about 1×10²-1×10³, 1×10²-1×10⁴, 1×10⁴-1×10⁵, or 1×10³-1×10⁶ PRPCs or photoreceptor cells. For example, the pharmaceutical preparation of PRPCs or photoreceptor cells may comprise at least about 20,000-200,000 PRPCs or photoreceptor cells in a volume at least of about 50-200 µL.

In the aforesaid pharmaceutical preparations and compositions, the number of PRPCs or photoreceptor cells or concentration of PRPCs or photoreceptor cells may be determined by counting viable cells and excluding non-viable cells. For example, non-viable PRPCs or photoreceptor may be detected by failure to exclude a vital dye (such as Trypan Blue), or using a functional assay (such as the ability to adhere to a culture substrate, phagocytosis, etc.). Additionally, the number of PRPCs or photoreceptor cells or concentration of PRPCs or photoreceptor cells may be determined by counting cells that express one or more PRPCs or photoreceptor cell markers and/or excluding cells that express one or more markers indicative of a cell type other than PRPCs or photoreceptor.

The PRPCs or photoreceptor cells may be formulated for delivery in a pharmaceutically acceptable ophthalmic vehicle, such that the preparation is maintained in contact with the ocular surface for a sufficient time period to allow the cells to penetrate the affected regions of the eye, as for example, the anterior chamber, posterior chamber, vitreous body, aqueous humor, vitreous humor, cornea, iris/ciliary, lens, choroid, retina, sclera, suprachoridal space, conjunctiva, subconjunctival space, episcleral space, intracorneal space, epicorneal space, pars plana, surgically-induced avascular regions, or the macula.

The PRPCs or photoreceptor cells may be contained in a sheet of cells. For example, a sheet of cells comprising PRPCs or photoreceptor cells may be prepared by culturing PRPCs or photoreceptor cells on a substrate from which an intact sheet of cells can be released, e.g., a thermoresponsive polymer such as a thermoresponsive poly(N-isopropylacrylamide) (PNIPAAm)-grafted surface, upon which cells adhere and proliferate at the culture temperature, and then upon a temperature shift, the surface characteristics are altered causing release the cultured sheet of cells (e.g., by cooling to below the lower critical solution temperature (LCST) (see da Silva et al., Trends Biotechnol. 2007 December; 25(12):577-83; Hsiue et al., Transplantation. 2006 Feb. 15; 81(3):473-6; Ide, T. et al. (2006); Biomaterials 27, 607-614, Sumide, T. et al. (2005), FASEB J. 20, 392-394; Nishida, K. et al. (2004), Transplantation 77, 379-385; and Nishida, K. et al. (2004), N. Engl. J. Med. 351, 1187-1196). The sheet of cells may be adherent to a substrate suitable for transplantation, such as a substrate that may dissolve in vivo when the sheet is transplanted into a host organism, e.g., prepared by culturing the cells on a substrate suitable for transplantation, or releasing the cells from another substrate (such as a thermoresponsive polymer) onto a substrate suitable for transplantation. An exemplary substrate potentially suitable for transplantation may comprise gelatin (see Hsiue et al., supra). Alternative substrates that may be suitable for transplantation include fibrin-based matrixes and others. The sheet of cells may be used in the manufacture of a medicament for the prevention or treatment of a disease of retinal degeneration. The sheet of PRPCs or photoreceptor cells may be formulated for introduction into the eye of a subject in need thereof. For example, the sheet of cells may be introduced into an eye in need thereof by subfoveal membranectomy with transplantation the sheet of PRPCs or photoreceptor cells, or may be used for the manufacture of a medicament for transplantation after subfoveal membranectomy.

The volume of preparation administered according to the methods described herein may be dependent on factors such as the mode of administration, number of PRPCs or photoreceptor cells, age and weight of the patient, and type and severity of the disease being treated. If administered by injection, the volume of a pharmaceutical preparations of PRPCs or photoreceptor cells of the disclosure may be from at least about 1, 1.5, 2, 2.5, 3, 4, or 5 mL, or more or less. The volume may be at least about 1-2 mL.

The method of treating retinal degeneration may further comprise administration of an immunosuppressant. Immunosuppressants that may be used include but are not limited to antilymphocyte globulin (ALG) polyclonal antibody, anti-thymocyte globulin (ATG) polyclonal antibody, azathioprine, BASILIXIMAB^{®} (anti-IL-2Rα receptor antibody), cyclosporin (cyclosporin A), DACLIZUMAB^{®} (anti-IL-2Rα receptor antibody), everolimus, mycophenolic acid, RITUXIMAB^{®} (anti-CD20 antibody), sirolimus, and tacrolimus. The immunosuppressants may be dosed at least about 1, 2, 4, 5, 6, 7, 8, 9, or 10 mg/kg. When immunosuppressants are used, they may be administered systemically or locally, and they may be administered prior to, concomitantly with, or following administration of the PRPCs or photoreceptor cells. Immunosuppressive therapy may continue for weeks, months, years, or indefinitely following administration of PRPCs or photoreceptor cells. For example, the patient may be administered 5 mg/kg cyclosporin for 6 weeks following administration of the PRPCs or photoreceptor cells.

The method of treatment of retinal degeneration may comprise the administration of a single dose of PRPCs or photoreceptor cells. Also, the methods of treatment described herein may comprise a course of therapy where PRPCs or photoreceptor cells are administered multiple times over some period. Exemplary courses of treatment may comprise weekly, biweekly, monthly, quarterly, biannually, or yearly treatments. Alternatively, treatment may proceed in phases whereby multiple doses are administered initially (e.g., daily doses for the first week), and subsequently fewer and less frequent doses are needed.

If administered by intraocular injection, the PRPCs or photoreceptor cells may be delivered one or more times periodically throughout the life of a patient. For example, the PRPCs or photoreceptor cells may be delivered once per year, once every 6-12 months, once every 3-6 months, once every 1-3 months, or once every 1-4 weeks. Alternatively, more frequent administration may be desirable for certain conditions or disorders. If administered by an implant or device, the PRPCs or photoreceptor cells may be administered one time, or one or more times periodically throughout the lifetime of the patient, as necessary for the particular patient and disorder or condition being treated. Similarly contemplated is a therapeutic regimen that changes over time. For example, more frequent treatment may be needed at the outset (e.g., daily or weekly treatment). Over time, as the patient's condition improves, less frequent treatment or even no further treatment may be needed.

The methods described herein may further comprise the step of monitoring the efficacy of treatment or prevention by measuring electroretinogram responses, optomotor acuity threshold, or luminance threshold in the subject. The method may also comprise monitoring the efficacy of treatment or prevention by monitoring immunogenicity of the cells or migration of the cells in the eye.

The PRPCs or PRs may be used in the manufacture of a medicament to treat retinal degeneration. The disclosure also encompasses the use of the preparation comprising PRPCs or PRs in the treatment of blindness. For example, the preparations comprising human PRPCs or PRs may be used to treat retinal degeneration associated with a number of vision-altering ailments that result in photoreceptor damage and blindness, such as, diabetic retinopathy, macular degeneration (including age related macular degeneration, e.g., wet age related macular degeneration and dry age related macular degeneration), retinitis pigmentosa, and Stargardt's Disease (fundus flavimaculatus), night blindness and color blindness. The preparation may comprise at least about 5,000-500,000 PRPCs or PRs (e.g., 100,00 PRPCs or PRs) which may be administered to the retina to treat retinal degeneration associated with a number of vision-altering ailments that result in photoreceptor damage and blindness, such as, diabetic retinopathy, macular degeneration (including age related macular degeneration), retinitis pigmentosa, and Stargardt's Disease (fundus flavimaculatus).

The PRPCs or PRs provided herein may be PRPCs or PRs. Note, however, that the human cells may be used in human patients, as well as in animal models or animal patients. For example, the human cells may be tested in mouse, rat, cat, dog, or non-human primate models of retinal degeneration. Additionally, the human cells may be used therapeutically to treat animals in need thereof, such as in veterinary medicine.

The following are examples to illustrate the disclosure and should not be viewed as limiting the scope of the disclosure.

### EXAMPLES

### Example 1: Materials and Methods

### Human induced pluripotent stem cell suspension culture

Human induced pluripotent stem cells (hiPSCs) used in this study were generated from human normal dermal fibroblast cells by using the StemRNA^{™}-NM Reprogramming kit (Stemgent, Cat # 00-0076). hiPSCs were routinely grown *in vitro* as colonies on 0.25µg/cm² iMatrix-511 Stem Cell Culture Substrate (Recombinant Laminin-511) (ReproCell) and cultured in NutriStem XF/FF^{™} Culture (Biological Industries). hiPSCs were transitioned from conventional 2D monolayer to 3D sphere culture in disposable spinner flasks (ReproCell) on a nine position stir plate (Dura-Mag, ChemCell) by dissociation with Accutase (Innovative Cell Technologies). Sphere adapted hPSCs were seeded as single cells at a density of 0.5-1 x 10⁶ cells / ml in 30 ml spinner flasks (ReproCell) containing culture medium (NutriStemO^{O} ) with 10 µM Y27632 (ReproCell). Agitation rates were adjusted to between 50-80 RPM depending on hiPSC lines. Medium was changed every day with fresh culture medium without Y27632, except for D1 after seeding when medium was only half changed. Spheres were dissociated with Accutase and/or TrypLE into single cells and passaged every 4-5 days when the sphere size reached approximately 230 - 260 µm. Cell sphere cultures were maintained in 5% CO₂ with 95% humidity at 37° C.

### Neural commitment and photoreceptor progenitor differentiation of 3D-hiPSC spheres

Following expansion for 3-5 passages in 3D culture in spinner flasks, dissociated 3D-hiPSC spheres were seeded at 1 x 10⁶ cell / ml. 24 hours later, undifferentiated hiPSC spheres were directly used for differentiation in spinner flasks with agitation speed at 50-80 RPM throughout the differentiation protocol. All media composition and factors are listed in Figure 2, panel A. In brief, cell spheres were first patterned at D0 with the dual-SMAD inhibitors SB431542 (1.5 to 15 µM, Reagents Direct) and LDN193189 (0.25 to 2.5 µM, ReproCell), and IFG1 (2.5 to 50 µg/ml, Peprotech). At D1, Wnt inhibitor IWR-1e (0.25 to 10 µM, Sigma) and Heparin (0.25 to 15 µg/ml, Sigma) were added to the differentiation induction medium. Heparin was withdrawn at D9 of neural commitment and IWR-1e at D11. All other factors were withdrawn at D15 of differentiation. For PRPC differentiation, from D2 to D13 by a gradual adaptation to NIM-3D medium through a dilution series of Pluriton^{™}/GF-free Nutristem and NIM-3D with the inducing factors mentioned above. From D18 to D27, spheres were adapted to PRPC-3D photoreceptor differentiation medium through a 50/50 adaptation containing NIM-3D/PRPC-3D medium. From D27, spheres were maintained in PRPC-3D medium. Medium was changed as follows: D0-D1: Pluriton^{™}/GF-free NutriStem^{®}:; D2-D5: 75% Pluriton^{™}/GF-free NutriStem^{®} -25% NIM-3D; D6-D9: 50% Pluriton^{™}/GF-free NutriStem^{®} -50% NIM-3D; D10-D13: Pluriton^{™}/GF-free NutriStem^{®} 25%-NIM-3D 75%; D13-D17: NIM-3D 100%; D18-D27: NIM-3D 50%-PRPC-3D 50%; from D27 on PRPC-3D 100%.

NIM-3D (Neural Induction Medium-3D) basal medium consisted of DMEM/F12 with HEPES, 1% N2 and 1% B27 serum-free supplements (Thermo Fisher Scientific), 1% penicillin/streptomycin, MEM Non-essential amino acids (Thermo Fisher Scientific), 0.30% glucose (Sigma) and all the factors described in Figure 2. PRPC-3D medium consisted of Neurobasal^{™} medium, 1% N2 and 1% B27 serum-free supplements (Thermo Fisher Scientific), 1% penicillin/streptomycin, MEM Non-essential amino acids (Thermo Fisher Scientific), 0.30% glucose (Sigma). Cells were incubated at 37°C with 5% CO₂. Approximately 85% of the medium was changed daily from D0 to D19 of neural differentiation and every 2-4 days after D19.

During differentiation, spheres were dissociated into single cells using TrypLE (Thermo Fisher Scientific) at different time points, and RNA was extracted for qRT-PCR analysis. Additional cells were processed for flow cytometry analysis and immunofluorescence staining. PRPCs were generated by continuous dissociation of spheres into single cells and reaggregation into 30 ml spinner flasks at D19, D30-D32, D50-D52, D80-82, when sphere diameter typically reached 350-450 µm to avoid generating hypoxic cells in the center of the spheres. Briefly, dissociation/reaggregation procedure consisted of collecting all spheres into 50ml conical tubes, followed by one wash with PBS and incubation with TrypLE for 30-60 minutes in a 37 °C water bath. Cells were triturated gently into single cells making sure it results into a homogeneous single cell suspension, followed by filtering through a 40 µm filter. Single cells were seeded into a 30ml spinner flask at a density of 0.5 - 1 x 10⁶ /ml in the appropriate culture medium with 10 µM Y27632. Morphology of the resulting spheres (size, appearance, and ability to incorporate into spheres) was monitored 2-3 days after each reaggregation and every week after that until next dissociation/reaggregation step.

For neural rosettes selection, an additional attachment of spheres/manual scraping step was introduced at D5/D12 and D11/D18 of neural differentiation. Briefly, 3ml of sphere suspension cultures at D5 and D11 were attached on 3 Matrigel (Corning) coated wells (of a 6-well plate) and maintained in the same medium used for continuous differentiation for an additional 7 days when neural rosettes formed in the center of the attached sphere. After one week in culture, at D12 and D18 respectively, cells were manually lifted with a scraper (Corning) and plated onto ultra-low attachment wells at 1:1 ratio to form spheres in suspension under static conditions. At D30-32 of differentiation, static spheres were dissociated into single cells and reaggregated into 30 ml spinner flasks and continued to be cultured using a similar protocol as our continuous dissociation/reaggregation procedure.

### Cryopreservation and thawing of early and late CRNPs and PRPCs

The hiPSC-derived retinal progenitors at various stages of *in vitro* differentiation (early CRNPs collected at D19, late CRNPs at D30-D34 and PRPCs at D50-D120), were harvested from dissociated suspension cultures of 30ml spinner flasks. Cells were dissociated into single cells and cryopreserved in Cryostor CS10 (Sigma) freeze medium supplemented with 10 µM Y-26632 at 40-50 million cells /vial for early and late CRNPs and at 5-10 million cells /vial for PRPCs in 1 ml aliquots using a rate controlled freezer. Cells were tested for recovery/ability to reaggregate in culture and viability following cryopreservation. Vials from 2 different differentiations were thawed and total viable cells were counted to determine recovery and viability. The average cell viability was about 80-90% at thaw and recovery was about 60- 80%. The thawed single cells into spinner flasks retained the ability to reaggregate, with sphere sizes ranging from 100 - 150 µm within 2-4 days post thawing which is comparable to those of continuous dissociation/reaggregation. Flow cytometric analysis of spheres at different days postthawing reveals a similar percentage of cells expressing PAX6/SOX2 compared to spheres formed post dissociation and reaggregation from the initial suspension culture. qPCR analysis also confirmed the expression of markers at similar levels, further demonstrating the feasibility of cryopreserving and banking large quantities of cells for future applications.

### Immunocytochemistry

PRPC spheres were dissociated into single cells using TrypLE and seeded on Matrigel coated 24-well plates *in vitro* at a 5.0 x10⁴ cells/well for 10-20 days in PRPC-3D medium. For D13 of differentiation, entire attached spheres were used for staining. Medium was removed, cells washed 3 times with DPBS with Ca/Mg (Thermo Fisher Scientific), and then fixed with 4% PFA (paraformaldehyde) (Electron Microscopy Sciences) for 15 minutes at room temperature followed by washing 3 times with DPBS. Cells were permeabilized and blocked with 5% normal donkey serum (NDS) (Jackson Immunolab) and 0.1% Triton X-100 (Sigma) in DPBS at room temperature for up to 1 hour, followed by incubation with primary antibodies diluted in blocking buffer overnight at 4°C. Primary antibodies and their dilution and source used for staining are summarized in Table 1. After overnight antibody incubation, the cells were washed 3 times with DPBS followed by subsequent incubation for 2 hours at room temperature under dark condition with the appropriate species specific fluorescently conjugated secondary antibodies diluted in DPBS containing 2.5% NDS and 0.1% Triton X-100: donkey anti-mouse Alexa Fluor^{®} 488- [1:1000] and donkey anti-rabbit Alexa Fluor^{®} 594-conjugated secondary antibodies [1:1000] (Thermo Fisher Scientific). Secondary antibodies used for immunostaining are listed in Table2. Cells were washed 3 times with DPBS and cell nuclei were counterstained with 1µg/ml 4',6-diamidino-2-phenylindole, dihydrochloride (DAPI) (Thermo Scientific) for 3 minutes at room temperature, followed by DPBS washing. Cells were examined using a computerassisted Nikon inverted microscope (Eclipse Ti-S) with a 4x, 10x and 20x objective, and images were captured and analyzed using NIS-Elements-BR software (Version 4.50, Nikon).

### Immunohistochemistry

D120 spheres were sectioned at 10-12 µm on a cryostat (Leica CM 1950) after embedding in OCT. compound (Scigen). For staining, slides stored at -80°C were allowed to air dry at room temperature for 1 hour, fixed in cold 4% PFA for 15 minutes, followed by washing 3 times with DPBS for 3 minutes each. Blocking and incubation with primary and secondary antibodies was performed as above directly on sections. Slides were mounted with Fluorogold-G containing DAPI (Southern Biotech) using coverslips, allowed to dry at room temperature and images acquired as described above.

### Flow cytometry analysis

Spheres were dissociated into single cells with TrypLE (Thermo Fisher Scientific), filtered through 40 µm strainer, and fixed with Fixation/Permeabilization buffer (BD Biosciences) for 12 minutes on ice. For flow cytometry using fluorescently conjugated antibodies to detect intracellular antigens, fixed 1.0-2.0 X 10⁵ cells / tube were permeabilized with ice-cold 1X BD Perm / Wash Buffer containing FBS and Saponin (BD Biosciences) for 30 minutes on ice, followed by incubation with appropriately conjugated antibodies (Summarized in Table 1) for 30 minutes under dark conditions. Cells were than washed with 2ml of Perm/Wash buffer and prepared for analysis. Control cells were incubated with mouse or rabbit IgG. For flow cytometry using unconjugated antibodies to detect intracellular antigens, fixed cells were blocked with blocking buffer consisting of 0.05% Triton X-100 (Sigma) and 5% normal donkey serum (NDS) (Jackson Immuno Research) in DPBS (Life Technologies) (Life technology) for 30 minutes on ice, followed by incubation with primary antibodies diluted in blocking buffer for 1 hour at room temperature, then washed with blocking buffer. Cells were then incubated with the appropriate donkey anti-rabbit Alexa Fluor^{®} 488 (Invitrogen) or donkey anti-mouse Alexa Fluor^{®} 647-conjugated secondary antibodies (Invitrogen) diluted in blocking buffer [1:1000] for 1 hour under dark conditions. After washing, cells were resuspended in blocking buffer. Control cells were incubated with secondary antibodies only. Cells were analyzed on an Accuri C6 flow cytometer (BD Biosciences) according to standard procedures. Data were analyzed with the BD Accuri C6 Plus software (BD).

### Quantitative Real-Time Polymerase Chain Reaction (qPCR)

Total RNA was isolated from cultured cells using the RNeasy Minikit (Qiagen), and concentration was measured using NanoDrop One (Thermo Scientific). qPCR was performed in a twostep reaction. For reverse transcription (RT), 0.5 µg of total RNA were transcribed to cDNA using the SuperScript OSM-IV VILO Master Mix cDNA Synthesis kit (Invitrogen) in accordance with the manufacturer's instructions, using a SympliAmp Thermal Cycler (Applied Biosystems). For qPCR reactions, 15 ng of cDNA was amplified in 20µl reaction mixtures containing TaqMan Gene Expression Assays and TaqMan Fast Advanced Master Mix (Applied Biosystems) using the QuantStudio^{™} 6 Flex Real-Time PCR System with 96-well plate block. All TaqMan Gene Expression Assays (TaqMan probes) used for the experiments are listed in Table 3. In all experiments, house-keeping gene GAPDH was used as an internal control for data normalization. Relative quantification data for each target gene was analyzed using the QuantStudio Real-Time PCR v1.3 software, based on 2^{(-ΔΔCT)} method [31] with iPSCs used as the reference control. Samples were done in triplicates and collected from 3 independent differentiations.

### Example 2: HiPSC sphere cultures

For a practical application of hPSCs in cell therapy, further refinement to large-scales and 3D culture systems are necessary. Towards this end, we have established a protocol to transit hiPSCs cultured in feeder-free 2D monolayer in NutriStem^{®} medium (**Figure 1****, panel A**) to a 3D dynamic suspension cultures, where hiPSCs were continuously cultured as uniform spheres in spinner flasks with NutriStem^{®} medium supplemented with small molecule Y27632 in the absence of feeder cells and matrix (**Figure 1****, panel B**) [25, 32-38]. With this suspension culture system, hPSC cultures can be serially passaged and consistently expanded for at least 10 passages. A typical passaging interval for 3D-hiPSC sphere is shown in **Figure 1****, panel B,** in which spheres with a size of 230-260 µm were dissociated into single cells using Accutase and reaggregated to reform spheres in spinner flasks under continuous agitation at 60-70RPM. Spheres gradually increased in size while maintaining a uniform structure together with a high pluripotency marker expression (**OCT4: 98.8%**) as demonstrated by flow cytometry analysis and a normal karyotype after 3-5 repeated passages. Our 3D-hPSC passaging method is broadly applicable, as it was successfully utilized for all routinely tested hPSC lines generated in our lab.

### Example 3: Efficient induction of retinal neuron differentiation of 3D hiPSC spheres with small molecules

To generate retinal neuronal progenitors at different developmental stages from hiPSCs, we developed a new approach in which 3D-hiPSC spheres in suspension were directly induced in a stepwise fashion with mainly small molecules (**Figure 2**). This new protocol in 3D spinner flasks integrates continuous 3D sphere culture with several dissociation/reaggregation steps with small molecules to induce retinal neuron differentiation. Instead of using protein factors for induction of hPSC differentiation toward retinal lineages as previously reported [2,8,10,11,16.21,22], we identified and used mainly small molecules, the quality of which can be easily controlled, to sequentially differentiate hPSC spheres to different developmental stages of retinal cells. Cells were first seeded as single cells (1 x 10⁶ cells / ml) in NutriStem^{®} medium supplemented with Y27632 in spinner flasks to form spheres. 24 hours later, designated as D0 of induction, hiPSC spheres were first treated with dual SMAD inhibitors SB431542 and LDN193189 to block the signal transduction of activin/transforming growth factor β (TGF-β) and bone morphogenetic protein (BMP) and to facilitate neural patterning [39], then Wnt inhibitor IWR-1e [10, 11, 24], IGF1 (an inducer of eye filed cell development) [18] and heparin were added to further induce retinal neural lineage commitment[16]. It has been shown that manipulating Wnt signaling with small molecule IWR-1e at early stages of neural induction has been reported to efficiently induce hPSCs to early photoreceptors progenitors in both adherent and retinal organoid suspension culture [23]. The sizes of spheres formed within 24 hours ranged from 100 µm to 150 µm, with optimal sizes range from 110 µm to 125 µm based on results from 4 hiPSC lines (**Figure 3B**). As differentiation progressed from D0 to D19, hiPSC spheres remained homogeneous in size while growing and expanding, and gradually acquiring a neural identity without dissociation. By D3-D5 of differentiation, spheres started to change their appearance with more irregular edges; by D19 these irregular edges disappeared completely, spheres became semitransparent and smooth, which are typical for neurospheres. During differentiation, we observed a gradual increase in cell numbers, ranging from 1.5 - 2.5 X 10⁸ cells (5 to 8 folds) at D9 when spheres reached sizes between 250-300 µm in diameter, and increased continually to approximately 3.0 - 4.5 X 10⁸ cells (10 to 15 folds, **Figure 3C**) at D19, when spheres reached about 400 µm in diameter (**Figure 3A**).

### Example 4: Kinetics analysis of retinal neuron differentiation from 3D-hPSC spheres

The kinetics of retinal differentiation and the efficiency of neural induction were examined by monitoring the appearance of Paired box 6 gene (PAX6) and Sex determining region Y-box 2 (SOX2) positive cells and PAX6/SOX2 double positive cells by flow cytometry analyses. The pluripotency octamer-binding transcription factor 4 gene (OCT4), known to be expressed at high levels in hPSCs, was detected at high levels at D0 prior to neural induction (98%), while gradually downregulated at D2 to about 50%, at D3 to about 5% and completely shut down at D4 to 0% during differentiation (**Figures 4A-4C**). SOX2, which is a marker for both pluripotent and neural stem cells was expressed at high levels both in hPSCs at D0 and cells following neural induction up to D19. The PAX6-positive and Pax6/SOX2- double positive cells, representing early committed retinal progenitor (CRNP) cells emerged at D3 (about 10%), and gradually upregulated between D3 to D4 (about 77%). Within the next 1-3 days at D5-D7 PAX6-positive and PAX6/SOX2-positive cells increased drastically (≥90%, **Figures 4A-4C**). 3D sphere cultures with at least about 90% of cells positive for PAX6 and PAX6/SOX2 were considered a successful induction.

It has been reported that different hPSC lines may vary in lineage specific differentiation and need optimization for conditions. Using the above protocol, we tested our neural differentiation conditions with 4 different hiPSC lines. In summary, each undifferentiated hPSC line was adapted to suspension cultures in 30ml spinner flaks and, spheres with a size range of about 100-150 µm were used for retinal neural differentiation with optimized concentrations of 10 µM SB431542, 1 µM LDN193189, 10ng/ml IGF1, IWR-1e (2 µM) and Heparin 2 µg/ml) at the timepoints indicated in **Figure 2****, panel A,** all hiPSC lines gave rise to PAX6+ and PAX6+/SOX2+ double positive cells (**Figure 4A**). We also noticed that hiPSC line #2 had a slightly different kinetics compared to other 3 lines, although OCT4 expression was completely shut down in a similar manner as the other lines, PAX6 population never reached > 90% at D5-D7 by flow cytometry analysis, indicating variability of neural induction among different cell lines is existed and the quality of different hiPSC lines needs to be tested before starting large scale cell production for the purpose of cell replacement therapy in the future. Nevertheless, these results demonstrated that our small molecule cocktail and neural inducing medium efficiently induced 3D-hPSC spheres towards neural lineage development continuously without disruption of the spheres.

To further examine the identity of cells derived from the above stepwise differentiation protocol, we seeded D11 spheres on Matrigel coated wells, cultured for an additional 2 days and examined the expression of PAX6, SOX2 and NESTIN (NES) as well as RAX1 which is a retina-specific transcription factor expressed in early retinal progenitors. These attached retinal spheres exhibit arrangement similar to neural rosettes and these cells are double positive for PAX6-RAX1 and NES-SOX2 (**Figure 5A**), further confirmed their retinal lineages. Quantitative real-time PCR gene expression analysis of spheres at different time point during earlier stages of differentiation showed that OCT4 gene was totally shut down at D5 (**Figure 5B**), confirming our flow cytometry results and further documenting the absence of PSCs in our 3D sphere culture. In contrast, the expression of neural marker PAX6 was elevated starting at D5 and continued in early stages of neural differentiation at D19, then gradually downregulated at D34-D40 (**Figure 5B**), coinciding with the onset of differentiation into more mature retinal cells. The expression of RAX1 at D5 indicated the appearance of early CRNPs, which was followed by high level expression of Ceh-10 homeodomain containing homolog (CHX10) gene at D13, a marker for retinal precursor cells, demonstrating the sequential appearance of different developmental retinal cell types in our 3D sphere cultures, recapitulating the temporal development observed in retinogenesis *in vivo.* Our results plus previous reports [9, 13, 40-42] showed that early retinal progenitor fate specification occurred at approximately 5 to 13 days in our 3D sphere culture system as illustrated in Figure 2. We named cells from D5 to D13 early **C**ommitted **R**etinal **N**euron **P**rogenitors (CRNP), and cells from D13 to D40 late CRNPs.

### Example 5: Generation of photoreceptor precursor cells from 3D CRNP spheres

While previous studies have reported the generation of photoreceptor progenitors from hPSCs with various degrees of efficiency in both 2D and 3D retinal organoids [9, 11, 15, 16, 18, 21, 43, 44], we also examined whether our differentiation protocol can efficiently generate postmitotic photoreceptor progenitors and photoreceptor-like cells. After the acquisition of early CRNP phenotype at about D19 **(****Figures 5A-5B****),** when the continuously differentiated 3D spheres reached about 400 µm in diameter, spheres were dissociated for the first time into single cells and reaggregated at cell densities of 0.5-1 x 10⁶ cells / ml in 30ml spinner flask with neural differentiation medium supplemented with Y27632 **(****Figure 6****)** to reform spheres. Immediate complete reaggregation of cells into spheres with morphological characteristics of neurospheres (phase contrast bright, semitransparent and have small microspikes on the periphery of the spheres) was achieved as early as one day after reaggregation with sphere size of about 110 µm (**Figure 6****, panel A**). Under differentiation conditions, gradual and continual sphere growth in size to about 400 µm was observed from about D14 to about D30, at this time spheres were dissociated again into single cells and reaggregated in spinner flasks. A similar dissociation/reaggregation approach was performed repeatedly at D50-52, D80-82, and D100-102 (**Figure 6****, panel A**). A comparable pattern of sphere formation and growth was observed during each dissociation/reaggregation cycle and approximately 100-fold increase in cell numbers was achieved from starting 3 X 10⁷ hiPSCs to about 3.0 - 4.5 X 10⁹ retinal neuron progenitors at about D100, which is far more efficient than previous reports (**Figure 6****, panel B**).

To examine the cellular composition of spheres, we seeded dissociated cells at dissociation/reaggregation timepoints of D32 and D82 (**Figure 7A**) and cultured *in vitro* for an additional about 1 to 3 weeks. Morphological characterization of attached single cells cultured under retinal differentiation conditions revealed neuronal connections resembling those of photoreceptor progenitors *in vitro* (**Figure 7A**). To further identify the real identity of these cells, the expression of several markers specific for photoreceptor progenitors were examined by immunofluorescence cytochemistry analyses. Our results clearly demonstrated that these cells expressed high levels of conerod homeobox (CRX), neural retina leucine zipper (NRL), and thyroid hormone receptor-β 2 (ThRB2), key transcription factors that are critical for photoreceptor fate specification and development at D100 of differentiation following several rounds of dissociation/reaggregation **(****Figure 7B****).** whereas only a small number of Ki67+ proliferating cells were detected in these spheres, indicating that postmitotic retinal neurons were efficiently generated in this 3D-sphere protocol. In addition, microtube associated protein (MAP2, green), a general marker for relative mature neuronal cells, was highly expressed, while glial fibrillary acidic protein (GFAP, red), a marker for astrocytes and/or Muller Glia, was sparsely detected in these cells, indicating a near homogeneous neuronal population expressing photoreceptor progenitor specific markers, we name these postmitotic retinal neurons **P**hoto**R**eceptor **P**recursor **C**ells (PRPC). We also examined the expression of mature photoreceptor cells markers at D100, and our results showed that most of these cells expressed rod visual pigment protein rhodopsin (RHOD) and neuronal calcium binding protein recoverin (REC), both are markers for rod photoreceptors **(****Figure 7C**).

Efficient differentiation towards PRPCs and photoreceptor-like cells was also confirmed by flow cytometry analyses at D80. Results showed that there was no detectable cell expressing the pluripotency gene OCT4, whereas over 90% of cells expressed PRPC and photoreceptor markers (CRX, 95.2%; NRL, 96.6%; NR2E3, 91.3%, REC, 96.8% and cone specific opsin red/green M-OPSIN, 91.2%, **Figure 8A**). To further characterize the expression kinetics of these genes during the differentiation process, we performed RT-qPCR analyses. These analyses further revealed a gradual increase of PRPC marker genes such as NRL, nuclear receptor subfamily 2, group E, member 3 (NR2E3), and ThRβ2 starting at D40 of differentiation (**Figure 8B**). Similarly, the expression kinetics of photoreceptor markers REC, RHOD and M-OPSIN showed the same trends, but the expression of M-OPSIN was only detectable at D70 (**Figure 8B**). Whereas PAX6, RAX1 and CHX10 genes, all are markers for early and late CRNP cells, were dramatically downregulated as shown in **Figure 5B****.** Concomitant down regulation of early retinal neuron genes and upregulation of late retinal neuron markers in these cells indicates these cells are at the developmental stages of PRPCs and photoreceptors. We therefore name cells differentiated more than 80 days (D80) photoreceptor-like cells. Same analyses were carried out with other 3 hiPSC lines (data not shown), demonstrating the repeatability and consistency of this 3D-sphere differentiation system. Karyotype analysis of hiPSC-derived PRPCs showed genetic stability following long term 3D sphere cultures by repeated dissociation/reaggregation steps (data not shown). Both spheres and single cells derived from these spheres were tested for viability after cryopreservation in liquid nitrogen, and ≈80% viable cells were recovered from cryopreserved early and late CRNPs and PRPCs (Data not shown)

To further characterize cells in the differentiated spheres, we embedded D 120 spheres in OCT and sectioned, general neuronal and retinal neuronal specific markers were examined by specific antibody staining (**Figure 9**). Qualitative assessment of spheres morphology and hematoxylin staining revealed clear cellular integrity throughout the entire sectional surface of the spheres without a necrotic core, further demonstrating that the spheres are supplied with proper oxygen and nutrients with metabolic wastes transported into the culture media during the extended suspension culture (**Figure 9**). High level expression of MAP2 throughout the section in a compact fashion (**Figure 9****, panel C**), together with high percentage of cells expressing PRPC specific markers, CRX and NRL, and very low number of Ki67+ proliferating cells further confirm the efficient generation of PRPCs in these spheres (Figure 9, panel D). More mature rod photoreceptors were abundant by D 120 as shown by wide-spread expression of RHOD and REC (Figure 10) in spheres organized within a recognizable structure of neuroepithelia. Together, these results demonstrate that our culture system supports the robust generation of large numbers of a highly pure and homogeneous PRPCs from hiPSCs by the combined effects of small molecules, dissociation/reaggregation and stimulated microgravity-enhanced microenvironment under continuous agitation.

**Table 1: List of primary unconjugated antibodies used for flow cytometry and immunofluorescence**

| **Antibody** | **Specie s** | **Catalog Number** | **Source** | **FC Dilution** | **IF Dilution** | **Marker** |
|---|---|---|---|---|---|---|
| **Beta III Tublin** | Mouse | ab78078 | Abcam | --- | 1:1000 | General immature neurons |
| **Cone Arrestin** | Rabbit | AB15282 | Millipore | 1:1000 | 1:300 | Cone photoreceptor progenitors |
| **CRX** | Mouse | H00001406 -M02 | Abnova | --- | 1:100 | Photoreceptor precursors |
| **CRX** | Rabbit | sc-30150(H-120) | Santa Cruz | 1:200 | --- | Photoreceptor precursors |
| **GFAP** | Rabbit | ab33922 | Abcam | --- | 1:500 | Astrocytes |
| **Ki67** | Rabbit | ab833 | Abcam | --- | 1:400 | Proliferating cells |
| **MAP2** | Mouse | 556320 | BD Pharmigen | --- | 1:1000 | General mature neurons |
| **Nestin** | Mouse | MAB1259 | R&D Systems | --- | 1:500 | CNS stem cells |
| **NR2E3** | Mouse | PP-H7223-00 | R&D Systems | --- | 1:100 | Rod photoreceptor progenitors |
| **NR2E3** | Rabbit | 14246-1-AP | Proteintech | --- | 1:500 | Rod photoreceptor progenitors |
| **NRL** | Rabbit | SAB110060 8 | Sigma | 1:2000 | 1:1000 | Early photoreceptor progenitors |
| **OCT4** | Rabbit | 2840 | Cell Signaling Technologies | 1:500 | 1:500 | Pluripotent cells |
| **Opsin-M** | Rabbit | AB5405 | Millipore | 1:1000 | 1:500 | Cone photoreceptor progenitors |
| **Pax6** | Mouse | | DSHB | 1:200 | 1:200 | Neural precursors |
| **PDE6 Alpha** | Rabbit | PA5-32974 | Thermofisher Scientific | --- | 1:200 | Mature rod photoreceptors |
| **RAX** | Rabbit | ab23340 | Abcam | 1:1000 | 1:100 | Eye field progenitors |
| **Recoverin** | Rabbit | AB5585 | Millipore | 1:2000 | 1:2000 | Mature rods and cone photoreceptors |
| **Rhodopsin** | Mouse | R5403 | Sigma | 1:200 | 1:2000 | Mature rod photoreceptors |
| **Sox2** | Rabbit | 3579 | Cell Signaling Technologies | 1:500 | 1:500 | Pluripotent cells, neural stem cells |
| **ThRB2** | Rabbit | ab53170 | Abcam | 1:2000 | 1:500 | Cone photoreceptor progenitors |
| **VSX2 (Chx10)** | Rabbit | HPA003436 | Sigma | --- | 1:100 | Retinal neural progenitors |

**Table 2: List of conjugated antibodies used for flow cytometry**

| **Antibody** | **Specie s** | **Catalog Number** | **Source** | **FC Dilution** |
|---|---|---|---|---|
| **Oct-4A (Alexa Fluor^{®} 488)** | Rabbit | 5177 | Cell Signaling Technologies | 1:200 |
| **Pax6 (Alexa Fluor 647)** | Mouse | 562249 | BD Biosciences | 1:200 |
| **Sox2 (Alexa Fluor 488)** | Rabbit | 5049 | Cell Signaling Technologies | 1:200 |

**Table3: List of TaqMan assays used for qPCR**

| **Gene name** | **TaqMan assay ID Number** |
|---|---|
| **ARR3** | Hs01020134_m1 |
| **ASCL1 (MASH1)** | Hs00269932_m1 |
| **CRX** | Hs00230899_m1 |
| **GAPDH** | Hs02786624_g1 |
| **NR2E3** | Hs00183915_m1 |
| **NRL** | Hs00172997_m1 |
| **OCT4** | Hs04260367_gH |
| **OPN1MW(Opsin-M)** | Hs04194752_g1 |
| **OPN1SW (Opsin-S)** | Hs00181790_m1 |
| **PAX6** | Hs01088114_m1 |
| **PDE6a** | Hs00166495_m1 |
| **RAX** | Hs00429459_m1 |
| **RCVRN (Recoverin)** | Hs00610056_m1 |
| **RHO (Rhodopsin)** | Hs00892431_m1 |
| **THRB (ThRB2)** | Hs00230861_m1 |
| **VSX2 (Chx10)** | Hs01584046_m1 |

### Example 6: Use of Sonic Hedgehog

Figure 11 is an overview of the neural induction protocol for the derivation of retinal neural progenitors from human induced pluripotent stem cells. The use of small molecules in combination with Sonic Hedgehog (SHH) efficiently generates retinal progenitor cells. Note that rh-SHH refers to recombinant human SHH.

Following expansion for 3-5 passages in 3D culture in spinner flasks, dissociated 3D-hiPSC spheres were seeded at 1 x 10⁶ cell / ml. 24 hours later, undifferentiated hiPSC spheres were directly used for differentiation in spinner flasks with agitation speed at 50-80 RPM throughout the differentiation protocol. All media composition and factors are listed in Figure 11. In brief, cell spheres were first patterned at D0 with the dual-SMAD inhibitors SB431542 ("SB", 1.5 to 15 µM, Reagents Direct) and LDN193189 ("LDN", 0.25 to 2.5 µM, ReproCell), and IFG1 (2.5 to 50 µg/ml, Peprotech). At D1, Wnt inhibitor IWR-1e (0.25 to 10 µM, Sigma) was added to the differentiation induction medium Pluriton^{™}. rh-SHH (0.5 to 20 nM, Sigma) was added at D3. rh-SHH was withdrawn at D9 of neural commitment and IWR-1e at D11. All other factors were withdrawn at D15 of differentiation. For PRPC differentiation, from D2 to D13 by a gradual adaptation to NIM-3D medium through a dilution series of Pluriton^{™}/GF-free NutriStem^{®} and NIM-3D with the inducing factors mentioned above. From D18 to D27, spheres were adapted to PRPC-3D photoreceptor differentiation medium through a 50/50 adaptation containing NIM-3D/PRPC-3D medium. From D27, spheres were maintained in PRPC-3D medium. Medium was changed as follows: DO-D1: Pluriton^{™}/GF-free NutriStem^{®}; D2-D5: 75% Pluriton^{™}/GF-free NutriStem^{®} -25% NIM-3D; D6-D9: 50% Pluriton^{™}/GF-free NutriStem^{®} -50% NIM-3D; D10-D12: Pluriton^{™}/GF-free NutriStem^{®} 25%-NIM-3D 75%; D13-D18: NIM-3D 100%; D19-D21: NIM-3D 50%-PRPM-3D 50%; from D22 on PRPM-3D 100%.

NIM-3D (Neural Induction Medium-3D) basal medium consisted of DMEM/F12 with HEPES, 1% N2 and 1% B27 serum-free supplements (Thermo Fisher Scientific), 1% penicillin/streptomycin, MEM Non-essential amino acids (Thermo Fisher Scientific), 0.30% glucose (Sigma) and all the factors described in Figure 11. PRPM-3D medium consisted of Neurobasal^{™} medium, 1% N2 and 1% B27 serum-free supplements (Thermo Fisher Scientific), 1% penicillin/streptomycin, MEM Non-essential amino acids (Thermo Fisher Scientific), 0.30% glucose (Sigma). Cells were incubated at 37°C with 5% CO₂. Approximately 85% of the medium was changed daily from D0 to D19 of neural differentiation and every 2-4 days after D19.

SHH-treated cultures exhibited a higher percentage of cells expressing PAX6, a transcription factor essential in retinal neurogenesis, in comparison to heparin-treated controls by day 7 of differentiation. At day 19, cultures treated with SHH demonstrated more than 10-fold increase in cells expressing PAX6 relative to controls. In contrast to the SHH conditions that showed a steady decrease in PAX6 expression from day 7 to 21, controls exhibited a steep decrease in PAX6 expressing cells by day 9, a possible indication that the cell populations have adopted alternative cell fates predominately from the Inner Nuclear Layer (INL) such as bipolar and amacrine cell types. Additionally, RT-PCR quantitation of neuronal and retinal specific markers in heparin-treated controls were less consistent across several independent rounds of retinal progenitor differentiation. These results indicate SHH is a more effective alternative to other mitogen-activated proteins such as heparin in achieving neuronal induction and the proliferation of retinal progenitors.

Figure 12 shows the comparative RT-PCR quantitation of PAX6 mRNA gene expression in differentiated cells treated with Heparin or SHH. Total RNA was collected from cells in both conditions during the photoreceptor differentiation timeline and analyzed for PAX6 RNA transcript levels.

The heparin-treated control group exhibited a slow and gradual increase in PAX6 mRNA gene expression between D0 to D20, indicating a less efficient hiPSC neural induction. On the contrary, the SHH-treated group demonstrated a significant and robust elevation in PAX6 RNA levels by day 5 and remained high till day 19 relative to controls. PAX6 gene expression levels for SHH-treated cells were more than 4-fold higher than cells from the control by day 5. These results suggest SHH is much more efficient than heparin at inducing hiPSCs to the neural lineage as well as maintaining neurogenesis of PAX6-positive cells during the first 19 days of differentiation *in vitro.*

### Example 7: A modified medium enhances maturation to a photoreceptor cell fate

To promote survival and maturation of precursor and early photoreceptor-like cells, we optimized our maturation medium to robustly specify rod and cone photoreceptor fates. At differentiation day 99, cell cultures were switched to medium containing 1% Glutamax, 1% Penicillin/streptomycin, human brain-derived neurotrophic factor (BDNF) (20 ng/mL), ascorbic acid (0.2 mM), and DAPT (1 µM) in Neurobasal^{™} medium. As shown in Figure 13, in comparison to controls, the above specified maturation medium increased rod (RHO) and cone (ThRβ2) markers by 20% and 10%, respectively. The photoreceptor precursor marker Recoverin was approximately 9% higher than controls. Fewer Nestin-positive cells were also observed in the modified maturation medium, indicating a greater majority of cells differentiated and exited the neural stem cell state. These immunocytochemical data suggests that the modified medium enhances maturation to a photoreceptor cell fate in comparison to control media.

### REFERENCES:

1. Stern, J.H., et al., Regenerating Eye Tissues to Preserve and Restore Vision. Cell Stem Cell, 2018. 22(6): p. 834-849.
2. Gonzalez-Cordero, A., et al., Recapitulation of Human Retinal Development from Human Pluripotent Stem Cells Generates Transplantable Populations of Cone Photoreceptors. Stem Cell Reports, 2017. 9(3): p. 820-837.
3. Santos-Ferreira, T.F., O. Borsch, and M. Ader, Rebuilding the Missing Part-A Review on Photoreceptor Transplantation. Front Syst Neurosci, 2016. 10: p. 105.
4. Schwartz, S.D., et al., Embryonic stem cell trials for macular degeneration: a preliminary report. Lancet, 2012. 379(9817): p. 713-20.
5. Schwartz, S.D., et al., Human embryonic stem cell-derived retinal pigment epithelium in patients with age-related macular degeneration and Stargardt's macular dystrophy: follow-up of two open-label phase 1/2 studies. Lancet, 2015. 385(9967): p. 509-16.
6. Schwartz, S.D., et al., Subretinal Transplantation of Embryonic Stem Cell-Derived Retinal Pigment Epithelium for the Treatment of Macular Degeneration: An Assessment at 4 Years. Invest Ophthalmol Vis Sci, 2016. 57(5): p. ORSFc1-9.
7. Zhao, C., Q. Wang, and S. Temple, Stem cell therapies for retinal diseases: recapitulating development to replace degenerated cells. Development, 2017. 144(8): p. 1368-1381.
8. Mellough, C.B., et al., Efficient stage-specific differentiation of human pluripotent stem cells toward retinal photoreceptor cells. Stem Cells, 2012. 30(4): p. 673-86.
9. Barnea-Cramer, A.O., et al., Function of human pluripotent stem cell-derived photoreceptor progenitors in blind mice, in Sci Rep. 2016. p. 29784.
10. Lamba, D.A., et al., Efficient generation of retinal progenitor cells from human embryonic stem cells. Proc Natl Acad Sci U S A, 2006. 103(34): p. 12769-74.
11. Lamba, D.A., et al., Generation, purification and transplantation of photoreceptors derived from human induced pluripotent stem cells. PLoS One, 2010. 5(1): p. e8763.
12. Phillips, M.J., et al., Modeling human retinal development with patient-specific induced pluripotent stem cells reveals multiple roles for visual system homeobox 2. Stem Cells, 2014. 32(6): p. 1480-92.
13. Zhu, J. and D.A. Lamba, Small Molecule-Based Retinal Differentiation of Human Embryonic Stem Cells and Induced Pluripotent Stem Cells. Bio Protoc, 2018. 8(12).
14. Lamba, D.A., M.O. Karl, and T.A. Reh, Strategies for retinal repair: cell replacement and regeneration. Prog Brain Res, 2009. 175: p. 23-31.
15. Reichman, S., et al., From confluent human iPS cells to self-forming neural retina and retinal pigmented epithelium. Proc Natl Acad Sci U S A, 2014. 111(23): p. 8518-23.
16. Nakano, T., et al., Self-formation of optic cups and storable stratified neural retina from human ESCs. Cell Stem Cell, 2012. 10(6): p. 771-85.
17. DiStefano, T., et al., Accelerated and Improved Differentiation of Retinal Organoids from Pluripotent Stem Cells in Rotating-Wall Vessel Bioreactors. Stem Cell Reports, 2018. 10(1): p. 300-313.
18. Mellough, C.B., et al., IGF-1 Signaling Plays an Important Role in the Formation of Three-Dimensional Laminated Neural Retina and Other Ocular Structures From Human Embryonic Stem Cells. Stem Cells, 2015. 33(8): p. 2416-30.
19. Meyer, J.S., et al., Optic vesicle-like structures derived from human pluripotent stem cells facilitate a customized approach to retinal disease treatment. Stem Cells, 2011. 29(8): p. 1206-18.
20. Ohlemacher, S.K., et al., Generation of highly enriched populations of optic vesicle-like retinal cells from human pluripotent stem cells. Curr Protoc Stem Cell Biol, 2015. 32: p. 1H 8 1-20.
21. Zhong, X., et al., Generation of three-dimensional retinal tissue with functional photoreceptors from human iPSCs. Nat Commun, 2014. 5: p. 4047.
22. Osakada, F., et al., Toward the generation of rod and cone photoreceptors from mouse, monkey and human embryonic stem cells. Nat Biotechnol, 2008. 26(2): p. 215-24.
23. Osakada, F., et al., Stepwise differentiation of pluripotent stem cells into retinal cells. Nat Protoc, 2009. 4(6): p. 811-24.
24. Osakada, F., et al., In vitro differentiation of retinal cells from human pluripotent stem cells by small-molecule induction. J Cell Sci, 2009. 122(Pt 17): p. 3169-79.
25. Pagliuca, F.W., et al., Generation of functional human pancreatic beta cells in vitro. Cell, 2014. 159(2): p. 428-39.
26. Chen, V.C., et al., Development of a scalable suspension culture for cardiac differentiation from human pluripotent stem cells. Stem Cell Res, 2015. 15(2): p. 365-75.
27. Kempf, H., et al., Cardiac differentiation of human pluripotent stem cells in scalable suspension culture. Nat Protoc, 2015. 10(9): p. 1345-61.
28. Kempf, H., et al., Controlling expansion and cardiomyogenic differentiation of human pluripotent stem cells in scalable suspension culture. Stem Cell Reports, 2014. 3(6): p. 1132-46.
29. Olmer, R., et al., Differentiation of Human Pluripotent Stem Cells into Functional Endothelial Cells in Scalable Suspension Culture. Stem Cell Reports, 2018. 10(5): p. 1657-1672.
30. Rigamonti, A., et al., Large-Scale Production of Mature Neurons from Human Pluripotent Stem Cells in a Three-Dimensional Suspension Culture System. Stem Cell Reports, 2016. 6(6): p. 993-1008.
31. Livak, K.J. and T.D. Schmittgen, Analysis of relative gene expression data using real-time quantitative PCR and the 2(-Delta Delta C(T)) Method. Methods, 2001. 25(4): p. 402-8.
32. Chen, V.C., et al., Scalable GMP compliant suspension culture system for human ES cells. Stem Cell Res, 2012. 8(3): p. 388-402.
33. Otsuji, T.G., et al., A 3D sphere culture system containing functional polymers for large-scale human pluripotent stem cell production. Stem Cell Reports, 2014. 2(5): p. 734-45.
34. Olmer, R., et al., Long term expansion of undifferentiated human iPS and ES cells in suspension culture using a defined medium. Stem Cell Res, 2010. 5(1): p. 51-64.
35. Lei, Y. and D.V. Schaffer, A fully defined and scalable 3D culture system for human pluripotent stem cell expansion and differentiation. Proc Natl Acad Sci U S A, 2013. 110(52): p. E5039-48.
36. Jiang, B., et al., Spheroidal formation preserves human stem cells for prolonged time under ambient conditions for facile storage and transportation. Biomaterials, 2017. 133: p. 275-286.
37. Wang, Y., et al., Scalable expansion of human induced pluripotent stem cells in the defined xeno-free E8 medium under adherent and suspension culture conditions. Stem Cell Res, 2013. 11(3): p. 1103-16.
38. Ismadi, M.Z., et al., Flow characterization of a spinner flask for induced pluripotent stem cell culture application. PLoS One, 2014. 9(10): p. e106493.
39. Chambers, S.M., et al., Highly efficient neural conversion of human ES and iPS cells by dual inhibition of SMAD signaling. Nat Biotechnol, 2009. 27(3): p. 275-80.
40. Meyer, J.S., et al., Modeling early retinal development with human embryonic and induced pluripotent stem cells. Proc Natl Acad Sci U S A, 2009. 106(39): p. 16698-703.
41. Zhu, J., et al., Immunosuppression via Loss of IL2rgamma Enhances Long-Term Functional Integration of hESC-Derived Photoreceptors in the Mouse Retina. Cell Stem Cell, 2017. 20(3): p. 374-384 e5.
42. Zhu, J., et al., Generation of Transplantable Retinal Photoreceptors from a Current Good Manufacturing Practice-Manufactured Human Induced Pluripotent Stem Cell Line. Stem Cells Transl Med, 2018. 7(2): p. 210-219.
43. Reichman, S. and O. Goureau, Production of Retinal Cells from Confluent Human iPS Cells. Methods Mol Biol, 2016. 1357: p. 339-51.
44. Reichman, S., et al., Generation of Storable Retinal Organoids and Retinal Pigmented Epithelium from Adherent Human iPS Cells in Xeno-Free and Feeder-Free Conditions. Stem Cells, 2017. 35(5): p. 1176-1188.
45. Reese, B.E., Development of the retina and optic pathway. Vision Res, 2011. 51(7): p. 613-32.
46. MacLaren, R., et al., Retinal repair by transplantation of photoreceptor precursors. Nature, 2006. 444:203-207.

## Claims

1. A method for in vitro production of photoreceptor precursor cells, comprising:
(a) 3-dimensional (3D) sphere culturing a plurality of pluripotent stem cells to generate a plurality of first spheres comprising eye early and late committed retinal neural progenitors (CRNPs);
(b) monitoring sphere size until the first spheres reach an average size of about 300-500 µm in diameter;
(c) disassociating the first spheres into a first plurality of substantially single cells;
(d) 3D sphere culturing the first plurality of substantially single cells to generate a plurality of second spheres comprising photoreceptor precursor cells (PRPCs);
(e) monitoring sphere size until the second spheres reach an average size of about 300-500 µm in diameter;
(f) disassociating the second spheres into a second plurality of substantially single cells;
(g) 3D sphere culturing the second plurality of substantially single cells to generate a plurality of third spheres comprising postmitotic PRPCs; and
(h) optionally, further differentiating the postmitotic PRPCs into photoreceptor-like cells.

2. The method of claim 1, wherein the pluripotent stem cells are embryonic stem cells or induced pluripotent stem cells, preferably from human.

3. The method of claim 1, wherein steps (a), (d) and (g) comprise culturing in a spinner flask or a stir-tank bioreactor, preferably under continuous agitation.

4. The method of claim 1, wherein step (a) further comprises gradually adapting to and culturing in a neural induction medium, preferably NIM-3D (Neural Induction Medium-3D) basal medium containing DMEM/F12 with HEPES, N2 and B27 serum-free supplements, penicillin/streptomycin, MEM non-essential amino acids, and glucose, supplemented with one or more of Sonic Hedgehog, Heparin, IWR-1e, SB431542, LDN193189 and IGF1.

5. The method of claim 4, further comprising providing SB431542, LDN193189 and IGF1 for a first period of time, providing IWR-1e for a second period of time that is shorter than the first period of time, and providing Sonic Hedgehog or Heparin for a third period of time that is shorter than the second period of time.

6. The method of claim 5, wherein the first period of time is 10-20 days, preferably 12-18 days, more preferably 16 days.

7. The method of claim 5, wherein the second period of time is 5-15 days, preferably 8-14 days, more preferably 11 days.

8. The method of claim 5, wherein the third period of time is 3-12 days, preferably 5-10 days, more preferably 7 days.

9. The method of claim 1, wherein in step (b) the first spheres reach an average size of about 350-450 µm in diameter.

10. The method of claim 1, wherein in step (b) the first spheres reach an average size of less than about 400 µm in diameter.

11. The method of claim 1, wherein steps (c) and (f) comprise contacting the first spheres and the second spheres, respectively, with a cell-dissociation enzyme.

12. The method of claim 1, wherein step (d) further comprises gradually adapting to and culturing in a photoreceptor differentiation medium, preferably PRPC-3D medium containing a serum-free basal neuronal cell culture medium, N2 and B27 serum-free supplements, penicillin/streptomycin, MEM non-essential amino acids, and glucose.

13. The method of claim 1, wherein step (g) and/or (h) further comprises switching to and culturing in a maturation medium, preferably a serum-free basal neuronal cell culture medium containing L-glutamine, Penicillin/streptomycin, human brain-derived neurotrophic factor (BDNF), ascorbic acid, and DAPT (N-[N-(3,5-difluorophenacetyl)-1-alanyl]-S-phenylglycine t-butyl ester).

14. The method of claim 1, wherein step (g) and/or (h) further comprises monitoring sphere size until about 300-500 µm in diameter; disassociating the third spheres into a third plurality of substantially single cells, preferably with a cell-dissociation enzyme; and reaggregating the third plurality of substantially single cells.

## Patentansprüche

1. Verfahren zur In-vitro-Herstellung von Photorezeptor-Vorläuferzellen, umfassend:
(a) dreidimensionale (3D-) Kugelkultur einer Vielzahl von pluripotenten Stammzellen, um eine Vielzahl von ersten Kugeln zu erzeugen, die frühe und späte determinierte Retinanervenvorläufer (CRNP) umfassen;
(b) Überwachen der Kugelgröße, bis die ersten Kugeln eine durchschnittliche Größe von etwa 300 bis 500 µm im Durchmesser erreichen;
(c) Trennen der ersten Kugeln in eine erste Vielzahl von im Wesentlichen einzelnen Zellen;
(d) 3D-Kultivieren der ersten Vielzahl von im Wesentlichen einzelnen Zellen, um eine Vielzahl von zweiten Kugeln, umfassend Photorezeptor-Vorläuferzellen (PRPC), zu erzeugen;
(e) Überwachen der Kugelgröße, bis die zweiten Kugeln eine durchschnittliche Größe von etwa 300 bis 500 µm im Durchmesser erreicht haben;
(f) Trennen der zweiten Kugeln in eine zweite Vielzahl von im Wesentlichen einzelnen Zellen;
(g) 3D-Kugelkultur der zweiten Vielzahl von im Wesentlichen einzelnen Zellen, um eine Vielzahl von dritten Kugeln, umfassend postmitotische PRPC, zu erzeugen; und
(h) gegebenenfalls weiteres Differenzieren der postmitotischen PRPC zu Photorezeptor-ähnlichen Zellen.

2. Verfahren nach Anspruch 1, wobei die pluripotenten Stammzellen embryonische Stammzellen oder induzierte pluripotente Stammzellen, vorzugsweise vom Menschen, sind.

3. Verfahren nach Anspruch 1, wobei die Schritte (a), (d) und (g) das Kultivieren in einer Spinnerflasche oder einem Rührtank-Bioreaktor, vorzugsweise unter kontinuierlichem Rühren, umfasst.

4. Verfahren nach Anspruch 1, wobei Schritt (a) weiters das schrittweise Anpassen an und Kultivieren in einem Nerveninduktionsmedium, vorzugsweise NIM-3D (Nerveninduktionsmedium-3D), Basalmedium, enthaltend DMEM/F12 mit serumfreien Ergänzungen HEPES, N2 und B27, Penicillin/Streptomycin, nichtessentielle MEM-Aminosäuren und Glucose, ergänzt mit einem oder mehreren aus Sonic Hedgehog, Heparin, IWR-1e, SB431542, LDN193189 und IGF1, umfasst.

5. Verfahren nach Anspruch 4, das weiters das Bereitstellen von SB431542, LDN193189 und IGF1 für einen ersten Zeitraum, das Bereitstellen von IWR-1e für einen zweiten Zeitraum, der kürzer ist als der erste Zeitraum, und das Bereitstellen von Sonic Hedgehog oder Heparin für einen dritten Zeitraum, der kürzer ist als der zweite Zeitraum, umfasst.

6. Verfahren nach Anspruch 5, wobei der erste Zeitraum 10 bis 20 Tage, vorzugsweise 12 bis 18 Tage, noch bevorzugter 16 Tage, beträgt.

7. Verfahren nach Anspruch 5, wobei der zweite Zeitraum 5 bis 15 Tage, vorzugsweise 8 bis 14 Tage, noch bevorzugter 11 Tage, beträgt.

8. Verfahren nach Anspruch 5, wobei der dritte Zeitraum 3 bis 12 Tage, vorzugsweise 5 bis 10 Tage, noch bevorzugter 7 Tage, beträgt.

9. Verfahren nach Anspruch 1, wobei in Schritt (b) die ersten Kugeln eine durchschnittliche Größe von etwa 350 bis 450 µm im Durchmesser erreichen.

10. Verfahren nach Anspruch 1, wobei in Schritt (b) die ersten Kugeln eine durchschnittliche Größe von weniger als etwa 400 µm im Durchmesser erreichen.

11. Verfahren nach Anspruch 1, wobei die Schritte (c) und (f) das Kontaktieren der ersten Kugeln bzw. der zweiten Kugeln mit einem Zelldissoziationsenzym umfassen.

12. Verfahren nach Anspruch 1, wobei Schritt (d) weiters das schrittweise Anpassen an und Kultivieren in einem Photorezeptordifferenzierungsmedium, vorzugsweise PRPC-3D-Medium, enthaltend ein serumfreies Basalnervenzellenkulturmedium, serumfreie N2- und B27-Ergänzungen, Penicillin/Streptomycin, nichtessentielle MEM-Aminosäuren und Glucose, umfasst.

13. Verfahren nach Anspruch 1, wobei Schritt (g) und/oder (h) weiters das Wechseln zu und Kultivieren in einem Reifungsmedium, vorzugsweise einem serumfreien Basalnervenzellenkulturmedium, enthaltend L-Glutamin, Penicillin/Streptomycin, menschlichen Hirn-abgeleiteten neutrotrophen Faktor (BDNF), Ascorbinsäure und DAPT (N-[N-(3,5-Difluorphenacetyl)-1-alanyl]-S-phenylglycin-t-butylester), umfasst.

14. Verfahren nach Anspruch 1, wobei Schritt (g) und/oder (h) weiters das Überwachen der Kugelgröße bis auf einen Durchmesser von etwa 300 bis 500 µm; Trennen der dritten Kugeln in eine dritte Vielzahl von im Wesentlichen einzelnen Zellen, vorzugsweise mit einem Zelldissoziationsenzym; und erneutes Aggregieren der dritten Vielzahl von im Wesentlichen einzelnen Zellen umfasst.

## Revendications

1. Procédé de production in vitro de cellules précurseurs de photorécepteurs, comprenant :
(a) la culture en sphères tridimensionnelles (3D) d'une pluralité de cellules souches pluripotentes pour générer une pluralité de premières sphères comprenant des progéniteurs neuronaux rétiniens compétents (CRNP) précoces et tardifs de l'œil ;
(b) la surveillance de la taille de sphères jusqu'à ce que les premières sphères atteignent une taille moyenne d'environ 300 à 500 µm de diamètre ;
(c) la dissociation des premières sphères en une première pluralité de cellules sensiblement uniques ;
(d) la culture en sphères 3D de la première pluralité de cellules sensiblement uniques pour générer une pluralité de deuxièmes sphères comprenant des cellules précurseurs de photorécepteurs (PRPC) ;
(e) la surveillance de la taille de sphères jusqu'à ce que les deuxièmes sphères atteignent une taille moyenne d'environ 300 à 500 µm de diamètre ;
(f) la dissociation des deuxièmes sphères en une deuxième pluralité de cellules sensiblement uniques ;
(g) la culture en sphères 3D de la deuxième pluralité de cellules sensiblement uniques pour générer une pluralité de troisièmes sphères comprenant des PRPC post-mitotiques ; et
(h) facultativement, la différenciation supplémentaire des PRPC post-mitotiques en cellules de type photorécepteur.

2. Procédé selon la revendication 1, dans lequel les cellules souches pluripotentes sont des cellules souches embryonnaires ou des cellules souches pluripotentes induites, de préférence d'origine humaine.

3. Procédé selon la revendication 1, dans lequel les étapes (a), (d) et (g) comprennent la culture dans un flacon centrifugeur ou un bioréacteur à cuve d'agitation, de préférence sous agitation continue.

4. Procédé selon la revendication 1, dans lequel l'étape (a) comprend en outre l'adaptation progressive à un milieu d'induction neuronale, et la culture dans celui-ci, de préférence un milieu basal NIM-3D (Neural Induction Medium-3D) contenant DMEM/F12 avec HEPES, des suppléments sans sérum N2 et B27, de la pénicilline/streptomycine, des acides aminés non essentiels MEM, et du glucose, complété à l'aide d'un ou plusieurs parmi Sonic Hedgehog, héparine, IWR-1e, SB431542, LDN193189 et IGF1.

5. Procédé selon la revendication 4, comprenant en outre la fourniture de SB431542, LDN193189 et IGF1 pendant une première période de temps, la fourniture d'IWR-1e pendant une deuxième période de temps qui est plus courte que la première période de temps, et la fourniture de Sonic Hedgehog ou d'héparine pendant une troisième période de temps qui est plus courte que la deuxième période de temps.

6. Procédé selon la revendication 5, dans lequel la première période de temps est de 10 à 20 jours, de préférence 12 à 18 jours, plus préférablement 16 jours.

7. Procédé selon la revendication 5, dans lequel la deuxième période de temps est de 5 à 15 jours, de préférence 8 à 14 jours, plus préférablement 11 jours.

8. Procédé selon la revendication 5, dans lequel la troisième période de temps est de 3 à 12 jours, de préférence 5 à 10 jours, plus préférablement 7 jours.

9. Procédé selon la revendication 1, dans lequel à l'étape (b), les premières sphères atteignent une taille moyenne d'environ 350 à 450 µm de diamètre.

10. Procédé selon la revendication 1, dans lequel à l'étape (b), les premières sphères atteignent une taille moyenne de moins d'environ 400 µm de diamètre.

11. Procédé selon la revendication 1, dans lequel les étapes (c) et (f) comprennent le contact des premières sphères et des deuxièmes sphères, respectivement, avec une enzyme de dissociation cellulaire.

12. Procédé selon la revendication 1, dans lequel l'étape (d) comprend en outre l'adaptation progressive à un milieu de différenciation de photorécepteurs, et la culture dans celui-ci, de préférence un milieu PRPC-3D contenant un milieu de culture cellulaire neuronale basal sans sérum, des suppléments sans sérum N2 et B27, de la pénicilline/streptomycine, des acides aminés non essentiels MEM, et du glucose.

13. Procédé selon la revendication 1, dans lequel l'étape (g) et/ou (h) comprend en outre la transition vers un milieu de maturation, et la culture dans celui-ci, de préférence un milieu de culture cellulaire neuronal basal sans sérum contenant de la L-glutamine, de la pénicilline/streptomycine, un facteur neurotrophique dérivé du cerveau humain (BDNF), de l'acide ascorbique, et DAPT (ester t-butylique de N-[N-(3,5-difluorophénacétyl)-1-alanyl]-S-phénylglycine).

14. Procédé selon la revendication 1, dans lequel l'étape (g) et/ou (h) comprend en outre la surveillance de la taille de sphères jusqu'à environ 300 à 500 µm de diamètre ; la dissociation des troisièmes sphères en une troisième pluralité de cellules sensiblement uniques, de préférence avec une enzyme de dissociation cellulaire ; et la réagrégation de la troisième pluralité de cellules sensiblement uniques.
